# EUROPEAN PATENT SPECIFICATION

(11) **EP 2 755 640 B1**
(45) Date of publication and mention of the grant of the patent: **26.07.2017**
(21) Application number: 12813950.8
(22) Date of filing: 14.09.2012
(51) Int. Cl.: A61K 9/16, A61K 31/485, A61K 9/20, A61K 9/48

(54) **TAMPER RESISTANT PHARMACEUTICAL FORMULATIONS**
MANIPULATIONSSICHERE PHARMAZEUTISCHE FORMULIERUNGEN
FORMULATIONS PHARMACEUTIQUES INVIOLABLES

(30) Priority: 16.09.2011 US 201161535743 P
(43) Date of publication of application: 23.07.2014
(73) Proprietor: Purdue Pharma LP, Stamford, CT 06901-3431 (US)
(72) Inventor: MCKENNA, William, Yonkers, NY 10704 (US); MULEY, Sheetal, R., Piscataway, NJ 08854 (US); ABU SHMEIS, Rama, Branchburg, NJ 08876 (US)
(74) Representative: Maiwald Patentanwalts GmbH
(86) International application number: PCT/IB2012/001914
(87) International publication number: WO 2013/038267

(56) References cited:
- EP-A1- 1 897 545
- WO-A1-2009/025859
- WO-A2-2006/058249
- WO-A2-2012/085656
- US-A1- 2005 031 546
- US-A1- 2008 063 725
- US-A1- 2008 152 595
- US-A1- 2008 254 112
- US-A1- 2009 022 798

## Description

### FIELD OF THE INVENTION

The present invention relates to the field of pharmaceutical dosage forms that are resistant to tampering and abuse.

### BACKGROUND

Pharmaceutical products are sometimes the subject of abuse. For example, a particular dose of opioid agonist may be more potent when administered parenterally as compared to the same dose administered orally. Some formulations can be tampered with to provide the opioid agonist contained therein for illicit use. Opioid agonist formulations intended for oral use are sometimes crushed or subject to extraction with solvents (e.g., ethanol) by drug abusers to provide the opioid contained therein for non-prescribed illicit use (e.g., nasal or parenteral administration).

There have previously been attempts in the art to control the abuse potential associated with opioid analgesics. For example, the combination of pentazocine and naloxone has been utilized in tablets available in the United States, commercially available as Talwin® Nx from Sanofi-Winthrop. Talwin® Nx contains pentazocine hydrochloride equivalent to 50 mg base and naloxone hydrochloride equivalent to 0.5 mg base. Talwin® Nx is indicated for the relief of moderate to severe pain. The amount of naloxone present in this combination has low activity when taken orally, and minimally interferes with the pharmacologic action of pentazocine. However, this amount of naloxone given parenterally has profound antagonistic action to narcotic analgesics. Thus, the inclusion of naloxone is intended to curb a form of misuse of oral pentazocine which occurs when the dosage form is solubilized and injected. Therefore, this dosage has lower potential for parenteral misuse than previous oral pentazocine formulations. A fixed combination therapy comprising tilidine (50 mg) and naloxone (4 mg) has been available in Germany for the management of severe pain since 1978 (Valoron® N, Goedecke). The rationale for the combination of these drugs is effective pain relief and the prevention of tilidine addiction through naloxone-induced antagonisms at the morphine receptor. A fixed combination of buprenorphine and naloxone was introduced in 1991 in New Zealand (Temgesic® Nx, Reckitt & Colman) for the treatment of pain.

Commonly owned U.S. Patent Application Publication No. 20090081290 is directed to opioid formulations that are resistant to crushing in attempts to liberate the drug contained therein for illicit use.

US 2009/022798 A1 relates to dosage forms and methods for the delivery of drugs, particularly drugs of abuse, characterized by resistance to solvent extraction; tampering, crushing or grinding, and providing an initial burst of release of drug followed by a prolonged period of controllable drug release.

WO 2006/058249 relates to a pharmaceutical composition (e.g., an oral solid pharmaceutical product) of any active drug substance susceptible to abuse, a gel forming polymer, a surfactant in sufficient amounts to cause nasal or mucosal irritation, and an agent in sufficient amounts to cause flushing, or other unpleasant peripheral vasodilatory effects, if the amount of the active drug subject to abuse is ingested in amounts exceeding the usual recommended therapeutic dose.

US 2005/031546 A1 relates to a dosage form containing, in addition to one or more active ingredients with abuse potential (A) optionally together with physiologically acceptable auxiliary substances (B), at least one synthetic or natural polymer (C) and optionally at least one wax (D), wherein component (C) exhibits a breaking strength of at least 500 N, and to a process for the production of the dosage form.

EP 1 897 545 A1 relates to tamper resistant solid oral extended release pharmaceutical dosage forms comprising an extended release matrix formulation.

US 2008/152595 A1 relates to a pharmaceutical composition (e. g. , an oral solid pharmaceutical product) of any active drug substance susceptible to abuse, a gel forming polymer, a surfactant in sufficient amounts to cause nasal or mucosal irritation, and an agent in sufficient amounts to cause flushing, or other unpleasant peripheral vasodilatory effects, if the amount of the active drug subject to abuse is ingested in amounts exceeding the usual recommended therapeutic dose.

WO 2009/025859 relates to a dosage form for sustained release of paliperidone, comprising at least a first component and second component, wherein the first component comprises at least one delay layer comprising a polymer, and the second component comprises non-coated Paliperidone and optionally comprises also coated Paliperidone; wherein the second component is located adjacent to the first component.

US 2008/063725 A1 relates to an oral pharmaceutical form comprising microparticles of reservoir type, with modified release of at least one AP, not subject to dose dumping in the presence of alcohol which resists immediate AP dose dumping in the presence of alcohol, in particular in a large volume and, in addition, the composition and the structure of which make it possible to prevent misuse of the AP this form contains, especially due to anti-misuse means.

WO 2012/085656 relates to a solid oral dosage form comprising (a) an inert tamper resistant core; and (b) a coating surrounding the core, the coating comprising an active agent.

US 2008/254112 A1 relates to a pharmaceutical active ingredient-containing formulation for oral administration which is coated with a single coating of a film-forming polymer, the coating comprising a mixture of at least two separating agents and no stabilizer.

There exists a need in the art for a dosage form containing a drug susceptible to abuse that is resistant to oral, parenteral and nasal abuse. In the case of opioid analgesics, there exists a need for a tamper resistant formulation that does not solely rely upon the inclusion of an antagonist in the formulation to deter abuse.

### SUMMARY OF THE INVENTION

It is an object of certain embodiments of the present invention to provide a solid oral dosage form comprising an active agent (e.g., an opioid analgesic) which is tamper resistant.

It is an object of certain embodiments of the present invention to provide a solid oral dosage form comprising an active agent (e.g., an opioid analgesic) which is subject to less oral abuse than other dosage forms.

It is an object of certain embodiments of the present invention to provide a solid oral dosage form comprising an active agent (e.g., an opioid analgesic) which is subject to less parenteral abuse than other dosage forms.

It is an object of certain embodiments of the present invention to provide a solid oral dosage form comprising an active agent (e.g., an opioid analgesic) which is subject to less intranasal abuse than other dosage forms.

It is a further object of certain embodiments of the present invention to provide a solid oral dosage form comprising an active agent (e.g., an opioid analgesic) which is subject to less diversion than other dosage forms.

It is a further object of certain embodiments of the present invention to provide a method of treating pain in human patients with a solid oral dosage form comprising an opioid analgesic while reducing the abuse potential of the dosage form.

It is a further object of certain embodiments of the present invention to provide a solid oral dosage form comprising an active agent (e.g., an opioid analgesic) which is resistant to dose dumping in the presence of alcohol.

It is another object of certain embodiments of the present invention to treat a disease or condition (e.g., pain) by administering a solid oral dosage form as disclosed herein to a patient in need thereof.

It is another object of certain embodiments of the present invention to provide a method of manufacturing an oral dosage form of an active agent (e.g., an opioid analgesic) as disclosed herein.

It is another object of certain embodiments of the present invention to provide a use of a medicament (e.g., an opioid analgesic) in the manufacture of a tamper-resistant dosage form as disclosed herein for the treatment of a disease state (e.g., pain).

The above objects of the present invention and others may be achieved by the present invention which is directed to an oral dosage form comprising from 2 to 75 particles, each particle comprising a compressed core comprising (i) an active agent susceptible to abuse, (ii) a gelling agent comprising polyethylene oxide; and (iii) a neutral acrylic polymer; wherein the 2 to 75 particles contain a therapeutically or prophylactically effective amount of the active agent; and wherein the viscosity resulting from mixing an intact unit dose of the dosage form with from 0.5 to 10 ml of an aqueous liquid is at least 10 mPa s (10 cP).

The compressed cores of the present invention may have a mean diameter from about 0.1 mm to about 10 mm; from about 0.5 mm to about 8 mm; from about 1 mm to about 6 mm; or from about 2 mm to about 4 mm.

In certain embodiments, the viscosity resulting from mixing an intact unit dose of the dosage form with from 0.5 to 10 ml of an aqueous liquid prevents or reduces absorption of the active agent by parenteral or nasal administration.

In certain embodiments, the amount of active agent released at 0.5 hour when measured in a USP Apparatus 1 (basket) at 100 rpm in 900 ml simulated gastric fluid (SGF) (with or without enzymes) with 40% ethanol at 37° C, is within 20% of the amount of active agent released at 0.5 hour when measured in a USP Apparatus 1 (basket) at 100 rpm in 900 ml simulated gastric fluid (SGF) (with or without enzymes) with 0% ethanol at 37°.

In certain embodiments, the dosage form is cured at a temperature greater than the glass transition temperature of the gelling agent for at least 1 minute.

In certain embodiments, the particles are combined with a diluent within a pharmaceutically acceptable capsule.

A process for preparing an oral dosage form comprising a plurality of particles may comprise preparing a plurality of compressed cores comprising (i) an active agent susceptible to abuse and (ii) a gelling agent; wherein the plurality of particles contains a therapeutically or prophylactically effective amount of the active agent; and wherein the viscosity resulting from mixing an intact unit dose of the dosage form with from 0.5 to 10 ml of an aqueous liquid prevents or reduces absorption of the active agent by parenteral or nasal administration.

A process for preparing an oral dosage form comprising a plurality of particles may comprise preparing a plurality of compressed cores comprising (i) an active agent susceptible to abuse and (ii) a gelling agent; wherein the plurality of particles contains a therapeutically or prophylactically effective amount of the active agent; and wherein the amount of active agent released at 0.5 hour when measured in a USP Apparatus 1 (basket) at 100 rpm in 900 ml simulated gastric fluid without enzymes (SGF) with 40% ethanol at 37° C, is within 20% of the amount of active agent released at 0.5 hour when measured in a USP Apparatus 1 (basket) at 100 rpm in 900 ml SGF with 0% ethanol at 37°C.

A process for preparing an oral dosage form comprising a plurality of particles may comprise preparing a plurality of compressed cores comprising (i) an active agent susceptible to abuse and (ii) a gelling agent; wherein the plurality of particles contains a therapeutically or prophylactically effective amount of the active agent; and wherein the dosage form is cured at a temperature greater than the glass transition temperature of the gelling agent for at least 1 minute.

A process for preparing an oral dosage form comprising a plurality of particles may comprise preparing a plurality of compressed cores comprising (i) an active agent susceptible to abuse and (ii) a gelling agent; wherein the plurality of particles contains a therapeutically or prophylactically effective amount of the active agent, wherein the plurality of particles are combined with a diluent within a pharmaceutically acceptable capsule.

A process for preparing an oral dosage form comprising a plurality of particles may comprise preparing a plurality of compressed cores comprising (i) an active agent susceptible to abuse and (ii) a gelling agent; wherein from 2 to 75 particles contains a therapeutically or prophylactically effective amount of the active agent.

A method of treating a disease or condition (e.g., pain, diarrhea or constipation) comprises administering to a patient in need thereof an oral dosage form as disclosed herein.

In describing the present invention, the following terms are to be used as indicated below. As used herein, the singular forms "a," "an," and "the" include plural references unless the context clearly indicates otherwise. Thus, for example, reference to "an active agent" includes a single active agent as well as a mixture of two or more different active agents, and reference to a "gelling agent" includes a single gelling agent as well as a mixture of two or more different gelling agents, and the like.

As used herein, the terms "active agent," "active ingredient," "pharmaceutical agent," and "drug" refer to any material that is intended to produce a therapeutic, prophylactic, or other intended effect, whether or not approved by a government agency for that purpose. These terms with respect to specific agents include all pharmaceutically active agents, all pharmaceutically acceptable salts thereof, and all complexes, stereoisomers, crystalline forms, cocrystals, ether, esters, hydrates and solvates thereof, and mixtures thereof.

As used herein, the terms "therapeutically effective" refers to the amount of drug or the rate of drug administration needed to produce a desired therapeutic result.

As used herein, the terms "prophylactically effective" refers to the amount of drug or the rate of drug administration needed to produce a desired prophylactic result.

As used herein, the term "stereoisomers" is a general term for all isomers of individual molecules that differ only in the orientation of their atoms in space. It includes enantiomers and isomers of compounds with one or more chiral centers that are not mirror images of one another (diastereomers).

The term "enantiomer" or "enantiomeric" refers to a molecule that is nonsuperimposable on its mirror image and hence optically active wherein the enantiomer rotates the plane of polarized light in one direction by a certain degree, and its mirror image rotates the plane of polarized light by the same degree but in the opposite direction.

The term "chiral center" refers to a carbon atom to which four different groups are attached.

The term "racemic" refers to a mixture of enantiomers.

The term "resolution" refers to the separation or concentration or depletion of one of the two enantiomeric forms of a molecule from a mixture.

The term "patient" means a subject who has presented a clinical manifestation of a particular symptom or symptoms suggesting the need for treatment, who is treated preventatively or prophylactically for a condition, or who has been diagnosed with a condition to be treated.

"Pharmaceutically acceptable salts" include, but are not limited to, inorganic acid salts such as hydrochloride, hydrobromide, sulfate, phosphate and the like; organic acid salts such as formate, acetate, trifluoroacetate, maleate, tartrate and the like; sulfonates such as methanesulfonate, benzenesulfonate, p-toluenesulfonate and the like; amino acid salts such as arginate, asparaginate, glutamate and the like; metal salts such as sodium salt, potassium salt, cesium salt and the like; alkaline earth metals such as calcium salt, magnesium salt and the like; and organic amine salts such as triethylamine salt, pyridine salt, picoline salt, ethanolamine salt, triethanolamine salt, discyclohexylamine salt, N,N'-dibenzylethylenediamine salt and the like.

The term "polyethylene oxide" is defined for purposes of the present invention as a composition of polyethylene oxide (PEO) without regard to molecular weight, and includes lower molecular weight PEOs usually referred to as polyethylene glycols. The term "high molecular weight polyethylene oxide (PEO)" is defined for proposes of the present invention as having an approximate molecular weight of at least 1,000,000, based on rheological measurements and the term "low molecular weight polyethylene oxide (PEO)" is defined for purposes of the present invention as having an approximate molecular weight of less than 1,000,000, based on rheological measurements.

The term "subject" is inclusive of the definition of the term "patient" and does not exclude individuals who are entirely normal in all respects or with respect to a particular condition.

The term "ppm" as used herein means "parts per million". Regarding 14-hydroxycodeinone, "ppm" means parts per million of 14-hydroxycodeinone in a particular sample product. The 14-hydroxycodeinone level can be determined by any method known in the art, preferably by HPLC analysis using UV detection.

### BRIEF DESCRIPTION OF THE DRAWINGS

Figures 1-5 are graphical depictions of the dissolution results of Example 1.
Figure 6 is a graphical depiction of the dissolution results of Example 2.
Figures 7-9 are graphical depictions of the dissolution results of Example 3.
Figures 10-11 are graphical depiction of the dissolution results of Example 4.
Figure 12 is a comparison of the dissolution results of Examples 4 and 5.
Figure 13 is a graphical depiction of the dissolution results of Example 5.
Figure 14 is a comparison of the dissolution results of Examples 4A, 6 and 7.
Figure 15 is a comparison of the dissolution results of Example 4A (first two granulation steps only), Example 4A (all three granulation steps) and Example 4B.

### DETAILED DESCRIPTION

The use of gelling agents has been contemplated in order to deter the abuse of dosage forms containing a drug susceptible to abuse (e.g., an opioid analgesic). One form of abuse is via the crushing of a dosage form in order to liberate the drug contained therein for illicit use such as parenteral administration or through absorption across an external mucosal surface. When the crushed dosage form is mixed with a solution, a viscosity is obtained which inhibits the active from being drawn into a needle to hinder parenteral abuse. Similarly, when the crushed dosage form is applied to a mucosal surface (e.g., the nasal cavity), the composition gels upon contact with mucosal moisture to inhibit absorption.

Controlled release oral dosage forms are sought out by abusers as the crushing of the dosage form may liberate an amount of active agent otherwise intended for prolonged release (e.g., 12 to 24 hours), making it immediately available. The immediate availability upon crushing may also make controlled release dosage forms more dangerous due to the possibility of accidental overdose.

Immediate release oral dosage forms are also the subject of abuse. For example, an oral dosage form may be crushed in order to make the drug therein available for administration by an unintended route, e.g., parenterally or nasally.

When formulating a tamper-resistant controlled or immediate release formulation, each one has obstacles to overcome in order to obtain a suitable product.

Controlled release formulations are sometimes formulated as multiparticulates in order to increase residence time in the gastrointestinal tract as compared to unitary dosage forms. However, a multiparticulate immediate release dosage form with a gelling agent to deter tampering may be difficult to obtain due to the inherent controlled release characteristics that the gelling agent may impart to the dosage form when included in sufficient amounts to inhibit tampering.

In certain situations, a dosage form can be abused without crushing by contacting the intact dosage form with a liquid to obtain dissolution of the active agent contained therein. This can be a particular issue with intact dosage forms that are in particulate form, given the larger surface areas and increased dissolution rate of such dosage forms.

Both controlled release and immediate release multiparticulate formulations may have formulation and pharmacokinetic issues such as (i) difficulty in manufacture, (ii) dose to dose variability in active agent, (iii) pharmacokinetic variability, (iv) variability due to administration with food, and (v) patient-to-patient variability which may be addressed by the present invention.

Immediate and controlled release dosage forms play a vital part in the management of both acute and chronic conditions (e.g., pain management with opioid analgesics). Therefore, it is important to provide a tamper-resistant dosage form of a drug susceptible to abuse that may be utilized for either controlled or immediate release to obtain a viable product that can provide effective plasma levels to a patient according to an intended release profile.

The present invention is directed to an oral dosage form comprising from 2 to 75 particles, each particle comprising a compressed core comprising:
(i) an active agent susceptible to abuse and
(ii) a gelling agent comprising polyethylene oxide; and
(iii) a neutral acrylic polymer
wherein the 2 to 75 particles contain a therapeutically or prophylactically effective amount of the active agent; and
wherein the viscosity resulting from mixing an intact unit dose of the dosage form with from 0.5 to 10 ml of an aqueous liquid is at least 10 mPa s (10 cP).

In certain embodiments, a viscosity that would prevent or reduce absorption is obtained when an intact unit dose of the dosage form is mixed with from 0.5 to 10 ml of an aqueous liquid.

In certain embodiments, the viscosity after mixing a dosage form (intact) with from 0.5 to 10 ml of an aqueous liquid is from 10 cP to 100 Cp; from 25 cP to 75 Cp; at least 20 cP; at least 40 cP or at least 60 cP. In other embodiments, the viscosity after mixing a dosage form (intact) with from 0.5 to 10 ml of an aqueous liquid is at least 10 cP, at least 25 cP, at least 75 Cp; at least 100 cP; at least 150 cP.

A unit dose of a dosage form of the present invention may include without limitation from 10 to 50 particles; from 15 to 25 particles; or from 10 to 50 particles. In certain embodiments of the present invention, each unit dose in a batch contains the same amount of particles. Such embodiments may be preferable to typical multiparticulate dosage forms which may contain a greater number of particles and may not uniformly contain the same amount of particulates in each unit dose. Further, typical multiparticulate dosage forms may not have content uniformity for each individual particle.

In certain embodiments, each particle contains a sub-therapeutic amount of active agent but collectively in a unit dosage form contain a therapeutic amount of the active agent.

Certain embodiments of the present invention may address formulation and pharmacokinetic issues such as (i) difficulty in manufacture, (ii) dose to dose variability in active agent, (iii) pharmacokinetic variability, (iv) variability due to administration with food, and (v) patient-to-patient variability.

The particles of the present invention may have a mean diameter from about 0.5 mm to about 10 mm; from about 1 mm to about 8 mm; from about 1.5 mm to about 6 mm; or from about 2 mm to about 4 mm.

### Gelling Agents

The gelling agent is included in the cores of the present invention in an amount to produce a sufficient viscosity to deter abuse after mixing the dosage form with an aqueous solution. The amount of gelling agent may also be selected in order to maintain or obtain a targeted release profile. In certain embodiments, the amount of gelling agent contained in the oral dosage forms of the present invention is not more than the amount of drug. In other embodiments, the amount of gelling agent contained in the oral dosage forms of the present invention is less than the amount of drug. In further embodiments, the amount of gelling agent contained in the oral dosage forms of the present invention is more than the amount of drug.

In certain embodiments, the oral dosage forms of the present invention contain a weight ratio of gelling agent to drug from about 20:1 to about 1:5; from about 10:1 to about 1:3; from about 5:1 to about 1:5; from about 3:1 to about 1:3; from about 1:1 to about 1:1.5; from about 1.5:1 to about 1:1; about 1:1.25; or about 1.25:1.

The gelling agent utilized in the oral dosage forms of the present invention comprises polyethylene oxide, and may further comprise an agent selected without limitation from sugars, sugar-derived alcohols (e.g., mannitol, sorbitol, and the like), starch and starch derivatives, cellulose derivatives (e.g., microcrystalline cellulose, sodium carboxymethyl cellulose, methylcellulose, ethyl cellulose, hydroxyethyl cellulose, hydroxypropyl cellulose, and hydroxypropyl methylcellulose), attapulgites, bentonites, dextrins, alginates, carrageenan, gum tragacanth, gum acacia, guar gum, xanthan gum, pectin, gelatin, kaolin, lecithin, magnesium aluminum silicate, alginic acid derivates (e.g., sodium and calcium salts of alginic acid), chitin derivatives (e.g., chitosan), carbomers, carbopols, polycarbophils, polyvinylpyrrolidone, polyethylene glycol, polyethylene oxide, polyvinyl alcohol, silicon dioxide, surfactants, mixed surfactant/wetting agent systems, emulsifiers, other polymeric materials, and mixtures thereof. In certain embodiments, the gelling agent further comprises xanthan gum. In other embodiments, the gelling agent further comprises pectin. The pectin or pectic substances include purified or isolated pectates and crude natural pectin sources such as from apple, citrus or sugar beet residues which have been subjected, when necessary, to esterification or de-esterification (e.g., by alkali or enzymes). The pectins may also be derived from citrus fruits such as lime, lemon, grapefruit, or orange. In preferred embodiments, the gelling agent further comprises an agent selected from, e.g., the group consisting of polyethylene oxide, hydroxypropylcellulose, hydroxyethylcellulose, hydroxypropylmethylcellulose, methylcellulose and mixtures thereof.

In embodiments comprising a low molecular weight polyethylene oxide, the low molecular weight polyethylene oxide can, for example, have an average molecular weight from about 10,000 to about 750,000 Daltons, or from about 50,000 to about 500,000 Daltons, or from about 75,000 to about 300,000 Daltons.

In embodiments comprising a high molecular weight polyethylene oxide, the high molecular weight polyethylene oxide can, for example, have an average molecular weight from about 1,000,000 to about 10,000,000 Daltons, or from about 2,000,000 to about 8,000,000 Daltons, or from about 4,000,000 to about 6,000,000 Daltons.

In certain embodiments, the oral dosage form comprises from about 5% (w/w) to about 95% (w/w) polyethylene oxide, or from about 10% (w/w) to about 75% (w/w) polyethylene oxide, or from about 15% (w/w) to about 40% (w/w) polyethylene oxide, or from about 20% (w/w) to about 30% (w/w) polyethylene oxide. In addition to polyethylene oxide, the present invention can include a nonionic triblock copolymer composed of a central hydrophobic chain of polyoxypropylene (poly(propylene oxide)) flanked by two hydrophilic chains of polyoxyethylene (poly (ethylene oxide)). These compounds are commercially available under the tradenames Lutrol® and Poloxamer®.

### Additional Excipients

The compressed cores of the present invention comprise a release modifying polymer which is a neutral acrylic polymer (e.g., Eudragit NE 30 D®, Eudragit NE 40 D® or Eudragit NM 30 D®), which can also provide crush-resistant characteristics to the dosage form.

The compressed cores of the present invention can include additional excipients in order to, e.g., aid manufacturing, provide additional tamper resistance, modify the release rate, or provide alcohol resistance.

The additional excipient may be at least one excipient selected from the group consisting of bulking agents, plasticizers, stabilizers, diluents, lubricants, binders, granulating aids, colorants, flavorants, and glidants.

In certain embodiments, the compressed core includes a further polymer that can modify the release rate of the active agent contained therein. Examples of polymers that can be utilized to modify the release of the active agent include pharmaceutically acceptable cellulosic polymers, including but not limited to cellulose esters, cellulose diesters, cellulose triesters, cellulose ethers, cellulose ester-ethers, cellulose acylates, cellulose diacylates, cellulose triacylates, cellulose acetates, cellulose diacetates, cellulose triacetates, cellulose acetate propionates, cellulose acetate butyrates and mixtures thereof. Preferably, the cellulosic polymer is an alkyl cellulosic polymer such as methylcellulose or ethylcellulose.
In other embodiments of the present invention, the further release modifying polymer is a pharmaceutically acceptable acrylic polymer selected without limitation from acrylic acid and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid) (anhydride), methyl methacrylate, polymethacrylate, poly(methyl methacrylate), poly(methyl methacrylate) copolymer, polyacrylamide, aminoalkyl methacrylate copolymer, poly(methacrylic acid anhydride), glycidyl methacrylate copolymers, and mixtures of any of the foregoing.

The active agent can be dry blended with the gelling agent and the optional additional excipient (e.g., neutral acrylic polymer) prior to being compressed into cores. In other embodiments the materials can be wet granulated, dried and optionally milled prior to being compressed into cores.

In certain embodiments, a portion or all of one or more of the active agent, gelling agent and optional additional excipient (e.g., neutral acrylic polymer) can be incorporated extra-granularly. For example, the active agent and the gelling agent can be wet granulated, dried and optionally milled. Thereafter, neutral acrylic polymer can be blended with the resultant granulation to obtain the active agent mixture to be compressed. Materials such as glidants and lubricants can also be added extragranularly in order to aid in manufacturing.

A release rate-modifying polymer can also be coated onto the compressed cores alternatively or in addition to inclusion of the polymer in the core. The coating can include one or more of the release modifying polymers as discussed above in an amount over the compressed cores to achieve a weight gain, e.g., from about 1% to about 30%, from about 2% to about 15% or from about 8% to about 12%.

Individual compressed cores can also include a film coating to enhance cosmetic appearance and/or to reduce tackiness. Examples of materials to be utilized as a film coat include hydroxypropylmethylcellulose, polyvinyl alcohol, lactose or a mixture thereof. The film coat can be (i) an outer coating directly coated onto a compressed core, (ii) an outer coating directly coated onto a compressed core along with a release-modifying coating, or (iii) an intermediate layer between a compressed core and a release modifying coating.

In certain embodiments, the cores of the oral dosage forms of the present invention comprise from about 0.5% (w/w) to about 80% (w/w) neutral acrylic polymer, or from about 1% (w/w) to about 60% (w/w) neutral acrylic polymer, or from about 5% (w/w) to about 50% (w/w) neutral acrylic polymer, or from about 10% (w/w) to about 40% (w/w) neutral acrylic polymer.

### Release Rates

The gelling agent and the release modifying material can be included in the dosage form to obtain an immediate or a controlled release dosage form.

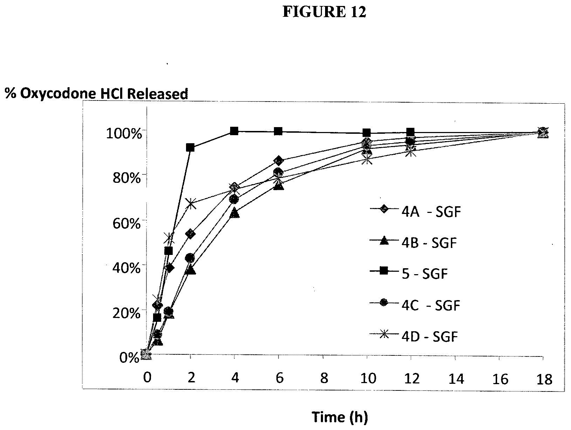

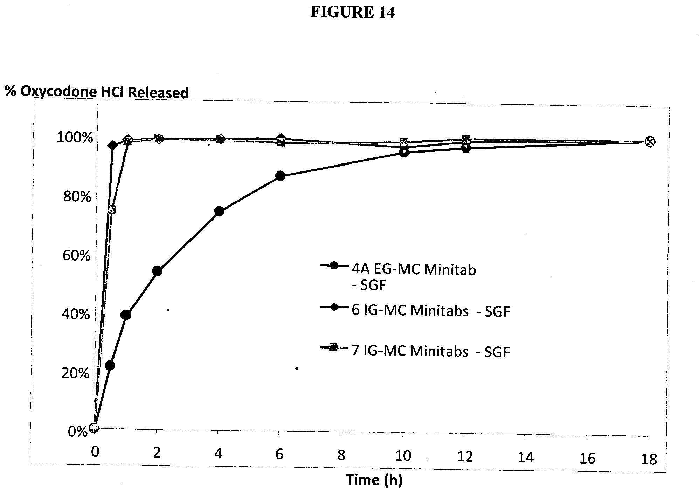

The process by which the release modifying polymer is incorporated into the core may impact the release profiles for the oral dosage form. For example, when the release modifying polymer is added through wet granulation with the active agent, the oral dosage form can exhibit a controlled release of the active agent. When the release modifying polymer is added via dissolution with the active agent, the oral dosage form can exhibit immediate release of the active agent. When the release modifying polymer is added via a combination of wet granulation and dissolution, the oral dosage form can exhibit biphasic release of the active agent. In addition, the addition of one release modifying polymer with the active agent prior to granulation with a second release modifying polymer (the same or different polymer) can exhibit immediate release of the active agent. Examples of these release profiles are shown in Figure 12, which depicts the release profiles of the formulations of Examples 4 and 5 below, and Figure 14 which depicts the release profiles of the formulations of Example 4A and 6 and 7 below.

In certain embodiments, dosage forms of the present invention provide an immediate release of the active agent such that at least about 85%, at least about 90%, or at least about 95% of the drug is released within about 45 minutes as measured by in-vitro dissolution in a USP Apparatus 1 (basket) at 100 rpm in 900 ml SGF (with or without enzymes) at 37° C.

In certain embodiments, dosage forms of the present invention provide an immediate release of the active agent such that at least about 90%, at least about 95%, or at least about 98% of the drug is released within about 60 minutes as measured by in-vitro dissolution in a USP Apparatus 1 (basket) at 100 rpm in 900 ml SGF (with or without enzymes) at 37° C.

In certain embodiments, dosage forms of the present invention provide a controlled release to provide, e.g., a 6-hour, or an 8-hour, or a 12-hour or a 24-hour formulation.

For 12 hour formulations, the dosage form can, e.g., provide a dissolution release rate in-vitro of the active agent (e.g. an opioid analgesic), when measured by the USP Basket Method at 100 rpm in 700 ml SGF (with or without enzymes) at 37° C of at least about 15% by weight of the active agent released at 1 hour and thereafter switching to 900 ml with Phosphate Buffer at a pH of 7.5 at 37° C, of from about 25% to about 65% by weight of the active agent released at 2 hours, from about 45% to about 85% by weight of the active agent released at 4 hours, and at least about 60% by weight of the active agent released at 8 hours.

For 24 hour formulations, the dosage form can, e.g., provide a dissolution release rate in-vitro of the active agent (e.g. an opioid analgesic), when measured by the USP Basket Method at 100 rpm in 700 ml SGF (with or without enzymes) at 37° C for 1 hour and thereafter switching to 900 ml with Phosphate Buffer at a pH of 7.5 at 37° C, of at least about 20% by weight of the active agent released at 4 hours, from about 20% to about 65% by weight of the active agent released at 8 hours, from about 45% to about 85% by weight of the active agent released at 12 hours, and at least about 80% by weight of the active agent released at 24 hours.

In certain embodiments, the amount of active agent (e.g. an opioid analgesic) released at 1 hour is from about 10%(w/w) to about 30%(w/w) as measured by an in-vitro dissolution in a USP Apparatus 1 (basket) at 100 rpm in 900 ml SGF (with or without enzymes) at 37° C.

In certain embodiments, the amount of active agent (e.g. an opioid analgesic) released at 2 hours is from about 25%(w/w) to about 50%(w/w) as measured by an in-vitro dissolution in a USP Apparatus 1 (basket) at 100 rpm in 900 ml SGF (with or without enzymes) at 37° C with a switch at 1 hour to 900 ml with Phosphate Buffer at a pH of 7.5 at 37° C.

In certain embodiments, the amount of active agent (e.g. an opioid analgesic) released at 4 hours is from about 40%(w/w) to about 80%(w/w) as measured by an in-vitro dissolution in a USP Apparatus 1 (basket) at 100 rpm in 900 ml SGF (with or without enzymes) at 37° C with a switch at 1 hour to 900 ml with Phosphate Buffer at a pH of 7.5 at 37° C.

In certain embodiments, the amount of active agent (e.g. an opioid analgesic) released at 8 hours is from about 65%(w/w) to about 95%(w/w) as measured by an in-vitro dissolution in a USP Apparatus 1 (basket) at 100 rpm in 900 ml SGF (with or without enzymes) at 37° C with a switch at 1 hour to 900 ml with Phosphate Buffer at a pH of 7.5 at 37° C.

In certain embodiments, the amount of active agent (e.g. an opioid analgesic) released at 12 hours is greater than about 80%(w/w) as measured by an in-vitro dissolution in a USP Apparatus 1 (basket) at 100 rpm in 900 ml SGF (with or without enzymes) at 37° C with a switch at 1 hour to 900 ml with Phosphate Buffer at a pH of 7.5 at 37° C.

In certain embodiments, the amount of active agent (e.g. an opioid analgesic) released at 1 hour is from about 15%(w/w) to about 25%(w/w) as measured by an in-vitro dissolution in a USP Apparatus 1 (basket) at 100 rpm in 900 ml SGF (with or without enzymes) at 37° C.

In certain embodiments, the amount of active agent (e.g. an opioid analgesic) released at 2 hours is from about 30%(w/w) to about 40%(w/w) as measured by an in-vitro dissolution in a USP Apparatus 1 (basket) at 100 rpm in 900 ml SGF (with or without enzymes) at 37° C with a switch at 1 hour to 900 ml with Phosphate Buffer at a pH of 7.5 at 37° C.

In certain embodiments, the amount of active agent (e.g. an opioid analgesic) released at 4 hours is from about 55%(w/w) to about 75%(w/w) as measured by an in-vitro dissolution in a USP Apparatus 1 (basket) at 100 rpm in 900 ml SGF (with or without enzymes) at 37° C with a switch at 1 hour to 900 ml with Phosphate Buffer at a pH of 7.5 at 37° C.

In certain embodiments, the amount of active agent (e.g. an opioid analgesic) released at 8 hours is from about 75%(w/w) to about 85%(w/w) as measured by an in-vitro dissolution in a USP Apparatus 1 (basket) at 100 rpm in 900 ml SGF (with or without enzymes) at 37° C with a switch at 1 hour to 900 ml with Phosphate Buffer at a pH of 7.5 at 37° C.

In certain embodiments, the amount of active agent (e.g. an opioid analgesic) released at 12 hours is greater than about 90%(w/w) as measured by an in-vitro dissolution in a USP Apparatus 1 (basket) at 100 rpm in 900 ml SGF (with or without enzymes) at 37° C with a switch at 1 hour to 900 ml with Phosphate Buffer at a pH of 7.5 at 37° C.

In certain embodiments, the amount of active agent (e.g. an opioid analgesic) released at 1 hour is from about 10%(w/w) to about 30%(w/w); the amount of active agent released at 2 hours is from about 25%(w/w) to about 50%(w/w); the amount of active agent released at 4 hours is from about 40%(w/w) to about 80%(w/w); the amount of active agent released at 8 hours is from about 65%(w/w) to about 95%(w/w), and the amount of active agent released at 12 hours is greater than about 80%(w/w); in each case, as measured by an in-vitro dissolution in a USP Apparatus 1 (basket) at 100 rpm in 900 ml simulated gastric fluid without enzymes (SGF) at 37° C.

In certain embodiments, the amount of active agent released at 1 hour is from about 15%(w/w) to about 25%(w/w); the amount of active agent released at 2 hours is from about 30%(w/w) to about 40%(w/w); the amount of active agent released at 4 hours is from about 55%(w/w) to about 75%(w/w); the amount of active agent released at 8 hours is from about 75%(w/w) to about 85%(w/w), and the amount of active agent released at 12 hours is greater than about 90%(w/w); in each case, as measured by an in-vitro dissolution in a USP Apparatus 1 (basket) at 100 rpm in 900 ml simulated gastric fluid without enzymes (SGF) at 37° C.

### Alcohol Resistance

The gelling agent and the release modifying agent can be selected in order to inhibit dose dumping of the active agent in the presence of alcohol. This characteristic is to prevent the dosage form from releasing the active agent at a rate faster than intended when alcohol is imbibed during residence of the dosage form in the gastrointestinal tract. Certain hydrophilic polymers (e.g., polyethylene oxide or methylcellulose) are suitable gelling agents that can provide alcohol resistance to the dosage form.

In certain embodiments, the amount of active agent (e.g. an opioid analgesic) released at 1 hour in 900 mL 0.1 N HCl (pH 1.5) with 40% EtOH using USP Apparatus II at 50 rpm is not more than the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 1 hour in 900 mL 0.1 N HCl (pH 1.5) with 40% EtOH using USP Apparatus II at 50 rpm is less than the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 1 hour in 900 mL 0.1 N HCl (pH 1.5) with 40% EtOH using USP Apparatus II at 50 rpm is within 25%(w/w) of the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 1 hour in 900 mL 0.1 N HCl (pH 1.5) with 40% EtOH using USP Apparatus II at 50 rpm is within 10%(w/w) of the amount of active agent released at 1 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is not more than the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is less than the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is within 25%(w/w) of the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 2 hour in 900 mL 0.1 N HCl (pH 1.5) with 40% EtOH using USP Apparatus II at 50 rpm is within 10%(w/w) of the amount of active agent released at 2 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is not more than the amount of active agent released at 4 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is less than the amount of active agent released at 4 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is within 25%(w/w) of the amount of active agent released at 4 hour in 900 mL 0.1 N HCl (pH 1.5) with 0% EtOH using USP Apparatus II at 50 rpm.

In certain embodiments, the amount of active agent (e.g. an opioid analgesic) released at 4 hour in 900 mL 0.1 N HCl (pH 1.5) with 40% EtOH using USP Apparatus II at 50 rpm is within 10%(w/w) of the amount of active agent released at 4 hour in 900 mL 0.1 N HCl (pH 1.5) with 0% EtOH using USP Apparatus II at 50 rpm.

### Tamper Resistance

In certain embodiments, the solid oral dosage form of the present invention demonstrates the tamper-resistant characteristic of not breaking or shattering when force is applied to it (by, for example, striking it with a hammer). Instead, the solid oral dosage form flattens without breaking or shattering. This characteristic makes it more difficult for the solid oral dosage form to be abused, by snorting the powder of a shattered tablet, chewing a tablet, or injecting a solution prepared from a shattered tablet. The inclusion of polyethylene oxide can provide tamper-resistant properties. The addition of neutral acrylic polymer also provides these properties.

In certain embodiments, the oral solid dosage form can be flattened without breaking, wherein the thickness of the dosage form after flattening corresponds to no more than about 60%, no more than about 50%, no more than about 40%, no more than about 30%, or no more than about 20% of the thickness of the dosage form before flattening.

In certain embodiments, the amount of active agent (e.g. an opioid analgesic) released at 0.5 hour from a flattened dosage form deviates no more than about 20%, no more than about 15%, or no more than about 10% points from a non-flattened dosage form as measured by an in-vitro dissolution in a USP Apparatus 1 (basket) at 100 rpm in 900 ml simulated gastric fluid without enzymes (SGF) at 37° C.

### Active Agents

In certain embodiments, the active agent used in the solid oral dosage form of the present invention is selected from the group consisting of ACE inhibitors, adenohypophoseal hormones, adrenergic neuron blocking agents, adrenocortical steroids, inhibitors of the biosynthesis of adrenocortical steroids, alpha-adrenergic agonists, alpha-adrenergic antagonists, selective alpha-two-adrenergic agonists, analgesics, anti-pyretics, anti-inflammatory agents, androgens, local and general anesthetics, anti-addictive agents, anti-androgens, anti-arrhythmic agents, antiasthmatic agents, anti-cholinergic agents, anti-cholinesterase agents, anti-coagulants, anti-diabetic agents, anti-diarrheal agents, anti-diuretics, anti-emetics, pro-kinetic agents, anti-epileptic agents, anti-estrogens, anti-fungal agents, anti-hypertensive agents, anti-microbial agents, anti-migraine agents, anti-muscarinic agents, antineoplastic agents, anti-parasitic agents, anti-parkinson's agents, anti-platelet agents, anti-progestins, anti-schizophrenia agents, anti-thyroid agents, anti-tussives, antiviral agents, atypical anti-depressants, azaspirodecanediones, barbiturates, benzodiazepines, benzothiadiazides, beta-adrenergic agonists, beta-adrenergic antagonists, selective beta-one-adrenergic antagonists, selective beta-two-adrenergic agonists, bile salts, agents affecting volume and composition of body fluids, butyrophenones, agents affecting calcification, calcium channel blockers, cardiovascular drugs, catecholamines and sympathomimetic drugs, cholinergic agonists, cholinesterase reactivators, contraceptive agents, dermatological agents, diphenylbutylpiperidines, diuretics, ergot alkaloids, estrogens, ganglionic blocking agents, ganglionic stimulating agents, hydantoins, agents for control of gastric acidity and treatment of peptic ulcers, hematopoietic agents, histamines, histamine antagonists, hormones, 5-hydroxytryptamine antagonists, drugs for the treatment of hyperlipoproteinemia, hypnotics, sedatives, immunosupressive agents, laxatives, methylxanthines, moncamine oxidase inhibitors, neuromuscular blocking agents, organic nitrates, opioid agonists, opioid antagonists, pancreatic enzymes, phenothiazines, progestins, prostaglandins, agents for the treatment of psychiatric disorders, retinoids, sodium channel blockers, agents for spasticity and acute muscle spasms, succinimides, testosterones, thioxanthines, thrombolytic agents, thyroid agents, tricyclic antidepressants, inhibitors of tubular transport of organic compounds, drugs affecting uterine motility, vasodilators, vitamins, and mixtures thereof.

In certain embodiments, the active agent is an opioid agonist. In such embodiments, the opioid agonist is selected from the group consisting of alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, pharmaceutically acceptable salts thereof, and mixtures thereof. In certain embodiments, the opioid agonist is selected from the group consisting of codeine, fentanyl, hydromorphone, hydrocodone, oxycodone, dihydrocodeine, dihydromorphine, morphine, tramadol, oxymorphone, pharmaceutically acceptable salts thereof, and mixture thereof.

In certain embodiments, the opioid agonist is oxycodone or a pharmaceutically acceptable salt thereof in an amount, e.g., from about 1 mg to about 200 mg. Specific amounts of oxycodone or a pharmaceutically acceptable salt thereof include about 2.5 mg, about 5 mg, about 7.5 mg, about 10 mg, about 15 mg, about 20 mg, about 30 mg, about 40 mg, about 60 mg, about 70 mg, about 80 mg, about 100 mg, about 120 mg or about 160 mg.

In certain embodiments of the present invention, the active agent is oxycodone hydrochloride having a 14-hydroxycodeinone level of less than about 25 ppm, less than about 15 ppm, less than about 10 ppm, less than about 5 ppm, less than about 2 ppm, less than about 1 ppm, less than about 0.5 ppm, or less than about 0.25 ppm.

WO 2005/097801 A1, U.S. Pat. No. 7,129,248 B2 and US 2006/0173029 A1 describe a process for preparing oxycodone hydrochloride having low levels of 14-hydroxycodeinone.

In certain embodiments, the solid oral dosage form of the present invention comprises an active agent that is an opioid antagonist (with or without an opioid agonist). In such embodiments, the opioid antagonist is selected from the group consisting of amiphenazole, naltrexone, methylnaltrexone, naloxone, nalbuphine, nalorphine, nalorphine dinicotinate, nalmefene, levallorphan, cyclazocine, pharmaceutically acceptable salts thereof and mixtures thereof.

In certain embodiments, the dosage forms of the present invention can include an opioid agonist and non-orally bioavailable opioid antagonist (e.g., naloxone). The non-orally bioavailable opioid antagonist can be contained within the compressed core or in a coating.

In certain embodiments, the solid oral dosage form of the present invention comprises an active agent that is a non-opioid analgesic. In such embodiments, the non-opioid analgesic is a non-steroidal anti-inflammatory agent selected from the group consisting of aspirin, celecoxib, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, pharmaceutically acceptable salts thereof and mixtures thereof.

In other embodiments, the present invention is directed to dosage forms utilizing active agents such as benzodiazepines, barbiturates or amphetamines, their antagonists, or combinations thereof.

Benzodiazepines to be used in the present invention include, but are not limited to alprazolam, bromazepam, chlordiazepoxide, clorazepate, diazepam, estazolam, flurazepam, halazepam, ketazolam, lorazepam, nitrazepam, oxazepam, prazepam, quazepam, temazepam, triazolam, pharmaceutically acceptable salts thereof and mixtures thereof. Benzodiazepine antagonists that can be used in the present invention include, but are not limited to, flumazenil and pharmaceutically acceptable salts thereof.

Barbiturates to be used in the present invention include, but are not limited to, amobarbital, aprobarbotal, butabarbital, butalbital, methohexital, mephobarbital, metharbital, pentobarbital, phenobarbital, secobarbital and pharmaceutically acceptable salts thereof and mixtures thereof. Barbiturate antagonists that can be used in the present invention include, but are not limited to, amphetamines and pharmaceutically acceptable salts thereof.

Stimulants to be used in the present invention include, but are not limited to, amphetamines, such as amphetamine, dextroamphetamine resin complex, dextroamphetamine, methamphetamine, methylphenidate, pharmaceutically acceptable salts thereof and mixtures thereof. Stimulant antagonists that can be used in the present invention include, but are not limited to, benzodiazepines, and pharmaceutically acceptable salts thereof.

### Methods of Manufacture

A process for preparing the immediate and controlled release oral dosage forms as disclosed herein comprising 2 to 75 particles, comprising a compressed core comprising (i) an active agent susceptible to abuse; (ii) a gelling agent comprising polyethylene oxide; and (iii) a neutral acrylic polymer; wherein the 2 to 75 particles contain a therapeutically or prophylactically effective amount of the active agent; and wherein the viscosity resulting from mixing an intact unit dose of the dosage form with from 0.5 to 10 ml of an aqueous liquid is at least 10 mPa s (10 cP), will be described below.

The process may include preparing an oral dosage form wherein the amount of active agent released at 0.5 hour when measured in a USP Apparatus 1 (basket) at 100 rpm in 900 ml simulated gastric fluid without enzymes (SGF) with 40% ethanol at 37° C, is within 20% of the amount of active agent released at 0.5 hour when measured in a USP Apparatus 1 (basket) at 100 rpm in 900 ml simulated gastric fluid without enzymes (SGF) with 0% ethanol at 37° C.

The process may include curing the compressed cores at a temperature greater than the glass transition temperature of the gelling agent for at least 1 minute.

The process may further include combining the 2 to 75 particles with a diluent within a pharmaceutically acceptable capsule.

The 2 to 75 particles can be further processed into a unit dosage form, e.g., by containment in a capsule, a sachet or a folded paper.

When the particles are contained in a capsule, certain embodiments include the co-containment of a diluent in the capsule along with the active agent particles. The incorporation of the diluent may serve to reduce agglomeration of the particles to facilitate the dosage form maintaining the targeted release profile.

The diluent can be mixed with the particles prior to incorporation in a capsule, or it can be added before or after incorporation of the active agent particles in the capsule.

In a preferred embodiment, the active agent particles are contained in the capsule and back-filled with the diluent.

The diluent can include without limitation, a saccharide (e.g., sucrose, dextrose, lactose, fructose, mannitol, and mixtures thereof), a polyethylene glycol or a cellulosic material (e.g., microcrystalline cellulose), or a mixture thereof.

### Other Formulations

The 2 to 75 particles can be in the form of a granulation, spheroids, compressed tablets, or beads and can have a mean diameter from about 0.1 mm to about 5 mm; from about 0.2 mm to about 3 mm; from about 0.5 mm to about 2.5 mm; or from about 1 mm to about 2 mm. The formulations can optionally be cured according to the parameters disclosed herein.

These embodiments preferably provide (i) a viscosity of the dosage form unsuitable for parenteral or nasal administration when mixed (intact) with from 0.5 to 10 ml of an aqueous liquid as disclosed herein, (ii) alcohol resistance as disclosed herein, and/or (iii) crush resistance as disclosed herein.

### Methods of Treatment

A method of treating a disease or condition comprises administering any of the tamper-resistant solid oral dosage forms described herein to a patient in need thereof. The patient may be treated e.g. for pain, diarrhea, or constipation.

The method may comprise administering the tamper-resistant solid oral dosage form described herein in combination with another pharmaceutical composition. The other pharmaceutical composition may be administered to treat the same condition or disease. The other pharmaceutical composition may also be administered to treat a different condition or disease.

The method of treatment may further comprise monitoring the patient for how the patient metabolizes the active agent, or how the patient responds to the active agent. The method of treatment further comprises altering the dose of the tamper-resistant solid oral dosage form in response to said monitoring. Certain baseline measurements are taken from the patient prior to administering the solid oral dosage form to the patient.

### Cured Formulations

A process for preparing the dosage form of the present invention may further comprise the step of curing the final dosage form. Curing is a process wherein the dosage form is subjected to certain conditions such as heat or electromagnetic radiation for a specified time in order to obtain a functional or physical change in the dosage form. The functional change can be the dosage form exhibiting a dissolution profile that does not change substantially over time. The physical change can, e.g., be the hardening of certain polymers (e.g., polyethylene oxides) that may be included in the dosage form.

The curing step may comprise at least partially melting the polyethylene oxide in the formulation. In particular, at least about 20%, or at least about 30%or at least about 40%, or at least about 50%, or at least about 60%, or at least about 75%, or at least about 90%, or about 100% of the polyethylene oxide in the formulation may melt during the curing step. Melting can be quantified by hot stage microscopy or differential scanning calorimetry.

The curing step may comprise subjecting the formulation to an elevated temperature for a certain period of time. The curing temperature may be at least as high as the softening temperature of the polyethylene oxide. The curing temperature may be at least about 60°C, at least about 62°C, ranges from about 62°C to about 90°C, from about 62°C to about 85°C, from about 62°C to about 80°C, from about 65°C to about 90°C, from about 65°C to about 85°C, or from about 65°C to about 80°C. The curing temperature preferably ranges from about 68°C to about 90°C, from about 68°C to about 85°C, from about 68°C to about 80°C, from about 70°C to about 90°C, from about 70°C to about 85°C, from about 70°C to about 80°C, from about 72°C to about 90°C, from about 72°C to about 85°C or from about 72°C to about 80°C. The curing temperature may be at least about 60°C or at least about 62°C, and less than about 90°C or less than about 80°C. Preferably, it is in the range of from about 62°C to about 72°C or from about 68°C to about 72°C. Preferably, the curing temperature is at least as high as the lower limit of the softening temperature range of the polyethylene oxide, or at least about 62°C, or at least about 68°C. The curing temperature may be at least as high as the upper limit of the softening temperature range of the polyethylene oxide, or at least about 72°C, or higher than the upper limit of the softening temperature range of the polyethylene oxide, or at least about 75°C, or at least about 80°C.

Where the curing step involves subjecting the formulation to an elevated temperature for a certain period of time, this period of time is hereinafter referred to as the curing time. For the measurement of the curing time, a starting point and an end point of the curing step are defined. The starting point of the curing step is defined to be the point in time when the curing temperature is reached.

The temperature profile during the curing step may show a plateau-like form between the starting point and the end point of the curing, wherein the end point of the curing step is defined to be the point in time when the heating is stopped or at least reduced, e.g. by terminating or reducing the heating and/or by starting a subsequent cooling step, such that the temperature subsequently drops below the curing temperature by more than about 10°C and/or below the lower limit of the softening temperature range of polyethylene oxide, for example, below about 62°C. When the curing temperature is reached and the curing step is thus started, deviations from the curing temperature in the course of the curing step can occur. Such deviations are tolerated as long as they do not exceed a value of about ± 10°C, preferably about ± 6°C, and more preferably about ± 3°C. For example, if a curing temperature of at least about 75°C is to be maintained, the measured temperature may temporarily increase to a value of about 85°C, about 81°C, or about 78°C, and the measured temperature may also temporarily drop down to a value of about 65°C, about 69°C or about 72°C. In the cases of a larger decrease of the temperature and/or in the case that the temperature drops below the lower limit of the softening temperature range of polyethylene oxide, for example below about 62°C, the curing step is discontinued, i.e. an end point is reached. Curing can be restarted by again reaching the curing temperature.

The temperature profile during the curing step may show a parabolic or triangular form between the starting point and the end point of the curing. This means that after the starting point, i.e., the point in time when the curing temperature is reached, the temperature further increases to reach a maximum, and then decreases, wherein the end point of the curing step is defined to be the point in time when the temperature drops below the curing temperature.

Depending on the apparatus used for the curing (i.e., curing device), different temperatures within the curing device can be measured to characterize the curing temperature.

The curing step may take place in an oven, wherein the temperature inside the oven is measured. Based thereon, when the curing step takes place in an oven, the curing temperature is defined to be the target inside temperature of the oven and the starting point of the curing step is defined to be the point in time when the inside temperature of the oven reaches the curing temperature. The end point of the curing step is defined to be (1) the point in time when the heating is stopped or at least reduced and the temperature inside the oven subsequently drops below the curing temperature by more than about 10°C and/or below the lower limit of the softening temperature range of the polyethylene oxide , for example below about 62°C, in a plateau-like temperature profile or (2) the point in time when the temperature inside the oven drops below the curing temperature in a parabolic or triangular temperature profile. Preferably, the curing step starts when the temperature inside the oven reaches a curing temperature of at least about 62°C, at least about 68°C, at least about 70°C, at least about 72°C or at least about 75°C. Preferably, the temperature profile during the curing step shows a plateau-like form, wherein the curing temperature, i.e. the inside temperature of the oven, is at least about 68°C, about 70°C, about 72°C, about 73°C, or lies within a range of from about 70°C to about 75°C, and the curing time is preferably in the range of from about 30 minutes to about 20 hours, from about 30 minutes to about 15 hours, from about 30 minutes to about 4 hours, or from about 30 minutes to about 2 hours, or from about 30 minutes to about 90 minutes.

The curing may take place in curing devices that are heated by an air flow and comprise a heated air supply (inlet) and an exhaust, e.g., a coating pan or fluidized bed. Such curing devices will hereinafter be called convection curing devices. In such curing devices, it is possible to measure the temperature of the inlet air, i.e., the temperature of the heated air entering the convection curing device and/or the temperature of the exhaust air, i.e., the temperature of the air leaving the convection curing device. It is also possible to determine or at least estimate the temperature of the formulations inside the convection curing device during the curing step, e.g., by using infrared temperature measurement instruments (such as an IR gun) or by measuring the temperature using a temperature probe that was placed inside the curing device near the formulations. Based thereon, when the curing step takes place in a convection curing device, the curing temperature can be defined and the curing time can be measured as follows.

In one process (method 1), the curing temperature is defined to be the target inlet air temperature and the starting point of the curing step is defined to be the point in time when the inlet air temperature reaches the curing temperature. The end point of the curing step is defined to be (1) the point in time when the heating is stopped or at least reduced and the inlet air temperature subsequently drops below the curing temperature by more than about 10°C and/or below the lower limit of the softening temperature range of the polyethylene oxide, for example below about 62°C, in a plateau-like temperature profile, or (2) the point in time when the inlet air temperature drops below the curing temperature in a parabolic or triangular temperature profile. Preferably, the curing step starts according to method 1, when the inlet air temperature reaches a curing temperature of at least about 62°C, at least about 68°C, at least about 70°C, at least about 72°C or at least about 75°C. Preferably, the temperature profile during the curing step shows a plateau-like form, wherein the curing temperature, i.e. the target inlet air temperature, is preferably at least about 72°C, for example, about 75°C, and the curing time which is measured according to method 1 is preferably in the range of from about 15 minutes to about 2 hours, for example, about 30 minutes or about 1 hour.

In another process (method 2), the curing temperature is defined to be the target exhaust air temperature, and the starting point of the curing step is defined to be the point in time when the exhaust air temperature reaches the curing temperature. The end point of the curing step is defined to be (1) the point in time when the heating is stopped or at least reduced and the exhaust air temperature subsequently drops below the curing temperature by more than about 10°C and/or below the lower limit of the softening temperature range of the polyethylene oxide, for example below about 62°C, in a plateau-like temperature profile, or (2) the point in time when the exhaust air temperature drops below the curing temperature in a parabolic or triangular temperature profile. Preferably, the curing step starts according to method 2, when the exhaust air temperature reaches a curing temperature of at least about 62°C, at least about 68°C, at least about 70°C, at least about 72°C or at least about 75°C. Preferably, the temperature profile during the curing step shows a plateau-like form, wherein the curing temperature, i.e. the target exhaust air temperature, is preferably at least about 68°C, at least about 70°C or at least about 72°C, for example the target exhaust air temperature is about 68°C, about 70°C, about 72°C, about 75°C or about 78°C, and the curing time which is measured according to method 2 is preferably in the range of from about 1 minute to about 2 hours or from about 5 minutes to about 90 minutes, for example, the curing time is about 5 minutes, about 10 minutes, about 15 minutes, about 30 minutes, about 60 minutes, about 70 minutes, about 75 minutes or about 90 minutes. More preferably, the curing time which is measured according to method 2 is in the range of from about 15 minutes to about 1 hour.

In a further process (method 3), the curing temperature is defined to be the target temperature of the formulations and the starting point of the curing step is defined to be the point in time when the temperature of the formulations, which can be measured for example by an IR gun, reaches the curing temperature. The end point of the curing step is defined to be (1) the point in time when the heating is stopped or at least reduced and the temperature of the formulations subsequently drops below the curing temperature by more than about 10°C and/or below the lower limit of the softening temperature range of the polyethylene oxide, for example below about 62°C, in a plateau-like temperature profile or (2) the point in time when the temperature of the formulations drops below the curing temperature in a parabolic or triangular temperature profile. Preferably, the curing step starts according to method 3, when the temperature of the formulations reaches a curing temperature of at least about 62°C, at least about 68°C, at least about 70°C, at least about 72°C or at least about 75°C.

In still another process (method 4), the curing temperature is defined to be the target temperature measured using a temperature probe, such as a wire thermocouple, that is placed inside the curing device near the formulations, and the starting point of the curing step is defined to be the point in time when the temperature measured using the temperature probe reaches the curing temperature. The end point of the curing step is defined to be (1) the point in time when the heating is stopped or at least reduced and the temperature measured using the temperature probe subsequently drops below the curing temperature by more than about 10°C and/or below the lower limit of the softening temperature range of polyethylene oxide, for example below about 62°C, in a plateau-like temperature profile, or (2) the point in time when the temperature measured using the temperature probe drops below the curing temperature in a parabolic or triangular temperature profile. Preferably, the curing step starts when the temperature measured using a temperature probe registers a temperature in the curing device of at least about 62°C, at least about 68°C, at least about 70°C, at least about 72°C or at least about 75°C. Preferably, the temperature profile during the curing step shows a plateau-like form, wherein the curing temperature is at least about 68°C, for example, about 70°C, and the curing time which is measured according to method 4 is preferably in the range of from about 15 minutes to about 2 hours or about 60 minutes or about 90 minutes.

If curing takes place in a convection curing device, the curing time can be measured by any of the methods described above.

The curing temperature may be defined as a target temperature range, for example as a target inlet air temperature range or a target exhaust air temperature range, wherein the starting point of the curing step is defined to be the point in time when the lower limit of the target temperature range is reached, and the end point of the curing step is defined to be the point in time when the heating is stopped or at least reduced, and the temperature subsequently drops below the lower limit of the target temperature range by more than about 10°C and/or below the lower limit of the softening temperature range of polyethylene oxide, for example, below about 62°C.

The curing time, i.e., the time period the formulation is subjected to the curing temperature, which can, for example, be measured according to the methods described above, is at least about 1 minute or at least about 5 minutes. The curing time may vary from about 1 minute to about 24 hours, from about 5 minutes to about 20 hours, from about 10 minutes to about 15 hours, from about 15 minutes to about 10 hours, or from about 30 minutes to about 5 hours depending on the specific formulation and the curing temperature. The curing time may vary from about 15 minutes to about 30 minutes. When the curing temperature is at least about 60°C, at least about 62°C, at least about 68°C, at least about 70°C, at least about 72°C or at least about 75°C, or varies from about 62°C to about 85°C or from about 65°C to about 85°C, then the curing time is preferably at least about 15 minutes, at least about 30 minutes, at least about 60 minutes, at least about 75 minutes, at least about 90 minutes or at least about 120 minutes. When the curing temperature is, for example, at least about 62°C, at least about 68°C, at least about 70°C, at least about 72°C or at least about 75°C, or ranges from about 62°C to about 80°C, from about 65°C to about 80°C, from about 68°C to about 80°C, from about 70°C to about 80°C or from about 72°C to about 80°C, then the curing time is preferably at least about 1 minute, at least about 5 minutes, at least about 10 minutes, at least about 15 minutes or at least about 30 minutes. The curing time can be chosen to be as short as possible while still achieving the desired result (e.g., increased tamper resistance). For example, the curing time preferably does not exceed about 5 hours, does not exceed about 3 hours or does not exceed about 2 hours. Preferably, the curing time is in the range of from about 1 minute to about 5 hours, from about 5 minutes to about 3 hours, from about 15 minutes to about 2 hours, or from about 15 minutes to about 1 hour.

The composition may be only subjected to the curing temperature until the polyethylene oxide present in the formulation has reached its softening temperature and/or at least partially melts. The curing time may be less than about 5 minutes, for example the curing time may vary from greater than 0 minutes to about 3 hours, from about 1 minute to about 2 hours or from about 2 minutes to about 1 hour. Instant curing is possible by choosing a curing device which allows for an instant heating of the polyethylene oxide in the formulation to at least its softening temperature, so that the polyethylene oxide at least partially melts. Such curing devices are, for example, microwave ovens, ultrasound devices, light irradiation apparatus such as UV-irradiation apparatus, ultra-high frequency (UHF) fields or any other apparatus known to the person skilled in the art.

The size of the formulation may determine the required curing time and curing temperature to achieve the desired tamper resistance.

The curing step may lead to a decrease in the density of the formulation, such that the density of the cured formulation is lower than the density of the formulation prior to the curing step. Preferably, the density of the cured formulation in comparison to the density of the uncured formulation decreases by at least about 0.5%. More preferably, the density of the cured formulation in comparison to the density of the uncured formulation decreases by at least about 0.7%, at least about 0.8%, at least about 1.0%, at least about 2.0% or at least about 2.5%.

In certain embodiments, the dosage form is cured at a temperature of at least the softening point of the polyethylene oxide for at least 1 minute, at least 5 minutes or at least 15 minutes.

In other embodiments, the dosage form is cured at a temperature of at least the softening point of the polyethylene oxide from about 1 minute to about 48 hours, from about 5 minutes to about 24 hours, from about 15 minutes to about 1 hour or about 30 minutes.

The dosage form can be cured, e.g., at a temperature of at least about 60° C, at least about 65° C, at least about 70° C, at least about 75° C or at a temperature of about 72° C.

In alternative embodiments, the dosage form can be cured, e.g., at a temperature from about 60° C to about 90° C, from about 62° C to about 72° C, from about 65° C to about 85° C, from about 70° C to about 80° C, from about 75° C to about 80° C or from about 70° C to about 75° C.

### Flattening Procedures

In certain embodiments, dosage forms of the present invention may be flattened without substantially compromising the release of the active agent or the integrity of the dosage form. Flatness is described in terms of the thickness of the smallest dimension of the flattened shape compared to the thickness of the smallest dimension of the non-flattened shape. This comparison is expressed in % thickness, based on either (i) the thickness of the smallest dimension of the non-flattened shape when the initial shape is non-spherical or (ii) the thickness of the diameter when the initial shape is spherical. The thickness may be measured using a thickness gauge (e.g., a digital thickness gauge or digital caliper). The flattening force may be applied by any possible method. For purposes of testing the dosage forms of the present invention, a carver style bench press may be used (unless otherwise specified) so as to achieve the target flatness or reduced thickness. According to certain embodiments of the invention, the flattening does not result in breaking of the dosage form into separate pieces; however, edge splits and cracks may occur.

In certain embodiments of the invention, a hammer can be used for flattening a dosage form. In such a process, hammer strikes can be manually applied from a direction substantially normal to the thickest dimension of the dosage form. The flatness is then described in the same manner as disclosed above.

In other embodiments, flattening can be measured relative to breaking strength or hardness tests, as described in Remington's Pharmaceutical Sciences, 18th edition, 1990, Chapter 89 "Oral Solid Dosage Forms", pages 1633-1665, using the Schleuniger Apparatus. In such an embodiment, the dosage form is pressed between a pair of flat plates arranged in parallel such that the force is applied substantially normal to the thickest dimension of the dosage form, thereby flattening the dosage form. The flattening of the dosage form may be described in terms of % flattening, based on the thickness of the dimension being flattened before conducting the breaking strength test. The breaking strength (or hardness) is defined as the force at which the tested dosage form breaks. Dosage forms that do not break, but which are deformed due to a force applied, are considered to be break-resistant at that particular force.

A further test to quantify the strength of dosage forms is the indentation test using a Texture Analyzer, such as the TA-XT2 Texture Analyzer (Texture Technologies Corp., 18 Fairview Road, Scarsdale, N.Y. 10583). In this method, a dosage form is placed on top of a stainless steel stand with a slightly concave surface and penetrated by the descending probe of the Texture Analyzer, such as a TA-8A 1/8 inch diameter stainless steel ball probe. Before starting the measurement, the dosage form is aligned directly under the probe, such that the descending probe will penetrate the tablet pivotally, i.e., in the center of the dosage form, and such that the force of the descending probe is applied substantially perpendicular to the diameter and substantially in line with the thickness of the dosage form. First, the probe of the Texture Analyzer starts to move towards the dosage form sample at the pre-test speed. When the probe contacts the dosage form surface and the trigger force set is reached, the probe continues its movement with the test speed and penetrates the dosage form. For each penetration depth or distance of the probe, the corresponding force is measured. When the probe has reached the desired maximum penetration depth, it changes direction and moves back at the post-test speed, while further measurements are taken. The cracking force is defined to be the force of the first local maximum that is reached in the corresponding force/distance diagram and is calculated using, for example, the Texture Analyzer software "Texture Expert Exceed, Version 2.64 English".

The following examples are set forth to assist in understanding the invention.

### Examples

### Reference Example 1

### Direct Compression Formulations

Cores comprising a gelling agent were prepared in accordance with Table 1:

**TABLE 1**

| **Ingredient** | **Amount (% w/w)** | | | | | | | |
|---|---|---|---|---|---|---|---|---|
| | **A1** | **A2** | **A3** | **A4** | **B1** | **B2** | **B3** | **B4** |
| Naltrexone HCI | 7.5 | 15 | 25 | 35 | 7.5 | 15 | 25 | 35 |
| PEO WSR 205 MW 600,000 | 92 | 84.5 | 74.5 | 64.5 | - | - | - | - |
| PEO WSR 303 MW 7,000,000 | - | - | - | - | 92 | 84.5 | 74.5 | 64.5 |
| Magnesium Stearate | 0.5 | 0.5 | 0.5 | 0.5 | 0.5 | 0.5 | 0.5 | 0.5 |
| Total | 100 | 100 | 100 | 100 | 100 | 100 | 100 | 100 |
| Dose per Capsule (mg) | 15 | 30 | 50 | 70 | 15 | 30 | 50 | 70 |

Processing of the cores included screening the polyethylene oxide through a screen with openings of ∼600 µm, followed by blending with naltrexone for 5 minutes in a Turbula mixer. This blend was then lubricated by blending with magnesium stearate (previously screened through a screen with openings of ∼600 µm) for 1 minute and directly compressing the blend with a Pressima with multi-tip punches and dies into tablets with a diameter of about 2 mm (yielding a tablet with about 5 mg total weight per single tablet) or with a diameter of about 4 mm (yielding a tablet with about 25 mg total weight per single tablet. Formulations A4 and B4 included 1% colloidal silicone dioxide to aid blend flow for tablet compression.

The tablets were cured at 70°C for 15 minutes in a tray dryer and filled into size 0 gelatin capsules to a fill weight of 200 ± 5 mg to obtain a unit dose of naltrexone HCl per capsule as described in Table 1 (approximately 40 of the 2 mm tablets or approximately 8 of the 4 mm tablets).

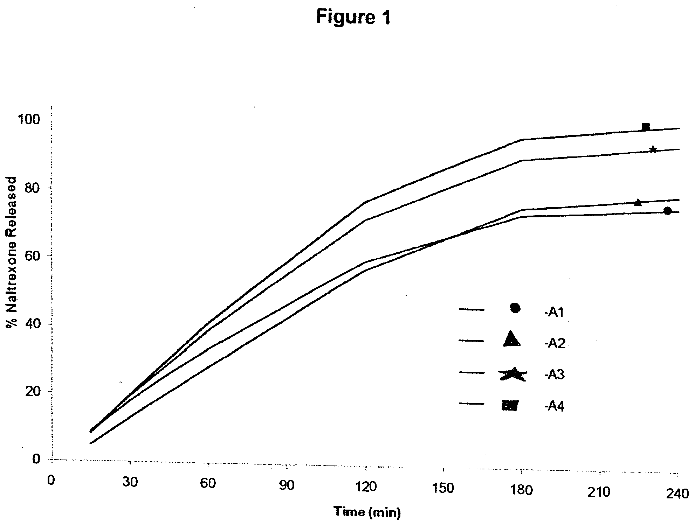

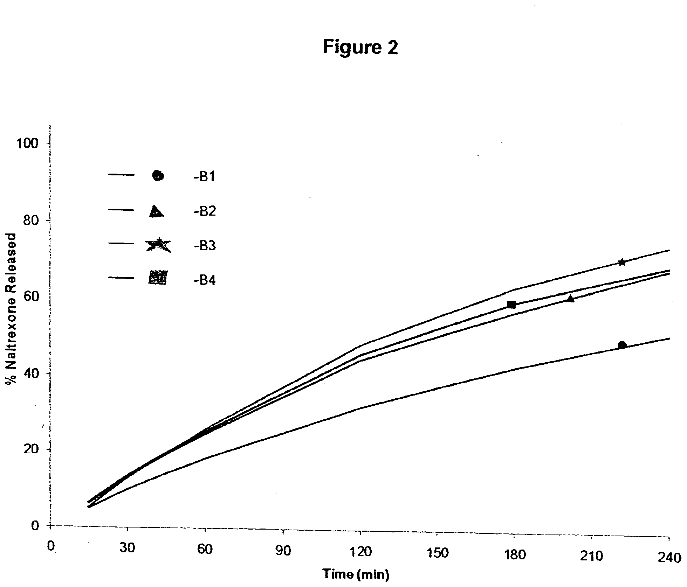

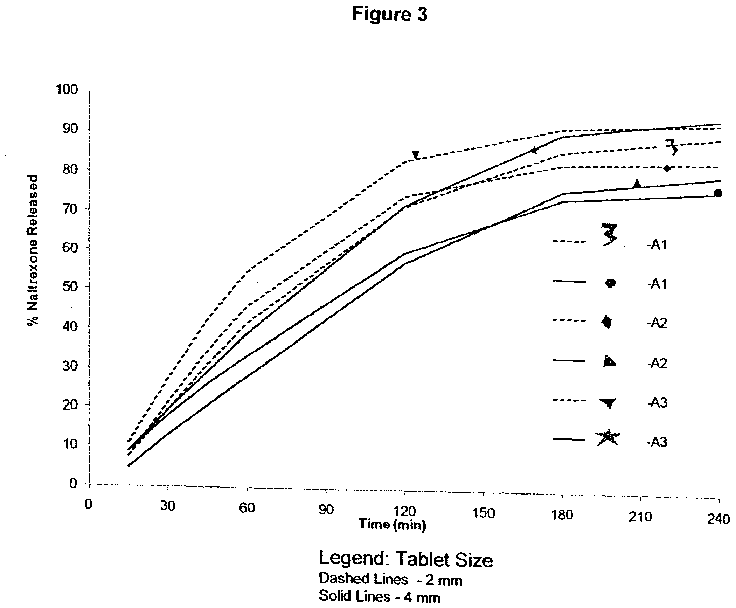

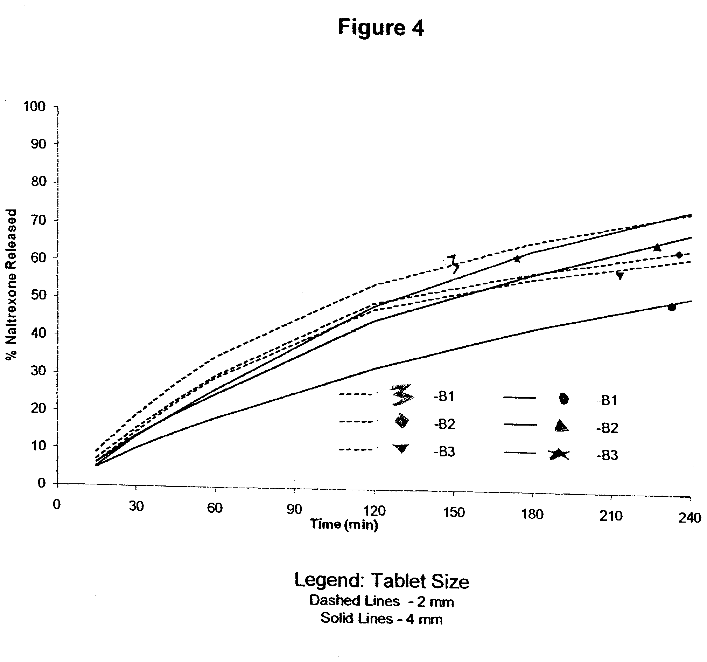

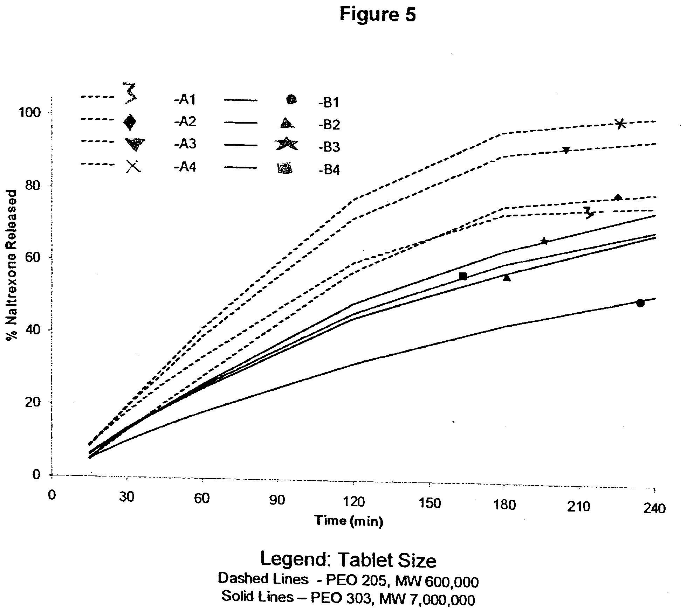

Formulations of Example 1 were subjected to dissolution testing in 900 mL simulated gastric fluid without enzymes at a pH of 1.2 using a USP Apparatus 1 with a #10 mesh basket at 100 rpm. The results are set forth in Figures 1-5.

### Example 2

### Naltrexone Wet Granulation Formulations

Cores comprising naltrexone, a gelling agent and a neutral acrylic polymer were prepared in accordance with Table 2:

**TABLE 2**

| **Ingredient** | **Amount (% w/w)** | | |
|---|---|---|---|
| | **2A** | **2B** | **2C** |
| Naltrexone HCI | 12.7 | 12.7 | 12.7 |
| PEO WSR 205 MW 600,000 | - | - | 84.8 |
| PEO WSR 303 MW 7,000,000 | 84.8 | 84.8 | - |
| Eudragit NE 40D | 1.5 | 1.5 | 1.5 |
| Magnesium Stearate | 1.0 | 1.0 | 1.0 |
| Total (g) | | | |
| Dose per Capsule (mg) | 25.34 | 25.34 | 25.34 |

Processing of the cores included wet granulating the naltrexone HCI with the Eudragit NE 40D without the addition of any filler or diluent. The wet granulation was performed by gradual addition of the Eudragit NE40D to the bowl of a high shear granulator (GMX Micro). The granules were dried in a tray dryer at ∼30 °C for 14-16 hours and dry milled using a Comil fitted with a screen containing round openings of about 0.040".

The polyethylene oxide was added as an extra-granular component. The blend was then lubricated by blending with magnesium stearate (previously screened through a screen with openings of ∼600 µm) for 1 minute and compressed on a Pressima tablet press using multi-tip punches and dies to obtain tablets of 3 mm diameter and average total weight of 13 mg.

The tablets were cured at 70°C for 15 minutes in a tray dryer and filled into size 0 gelatin capsules at a fill weight of 200 ± 5 mg to obtain a dose of 25.34 mg naltrexone HCl per capsule (approximately 16 of the 3 mm tablets).

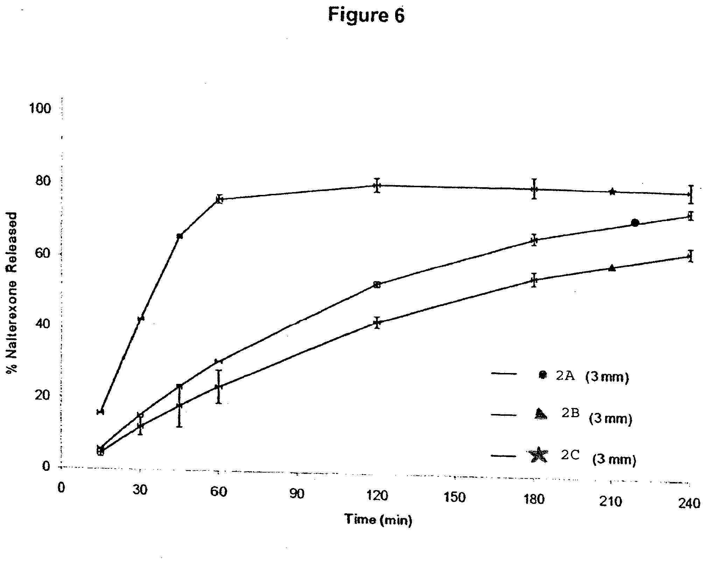

Formulations of Example 2 were subjected to dissolution testing in simulated gastric fluid without enzymes at a pH of 1.2 using a USP Apparatus 1 with a #10 mesh basket at 100 rpm. The results are set forth in Figure 6.

### Example 3

### Oxycodone Wet Granulation Formulations

Cores comprising oxycodone, a gelling agent and a neutral acrylic polymer were prepared in accordance with Table 3:

**TABLE 3**

| **Portion** | **Ingredient** | **Amount (% w/w)** | |
|---|---|---|---|
| | | **3A** | **3B** |
| Intragranular | Oxy HCl | 16.7 | 17.4 |
| | Microcrystalline cellulose (MCC) | 30.2 | 21.7 |
| | Eudragit-NE solids | 31.3 | 26.1 |
| | Intragranular Total | 78.2 | 65.5 |
| Extragranular | PEO WSR 205 | 20.8 | 33.7 |
| | Magnesium Stearate | 0.5 | 0.55 |
| | Collodial Silicon Dioxide | 0.5 | 0.55 |
| | Total | 100 | 100 |

Processing of the cores included wet granulation of oxycodone HCl and microcrystalline cellulose with Eudragit NE 40D aqueous dispersion. The Eudragit® NE40D aqueous disperion was incorporated into the granulation in two steps to increase the amount of solid NE. In formulation 3A, 62.5% of the total Eudragit NE was incorporated in the first granulation step, and the remainder 37.5% was incorporated in the second granulation step. In formulation 3B, 50% of the total Eudragit NE was incorporated in the first granulation step, and the remainder 50% was incorporated in the second granulation step.

Each step of the wet granulation was performed by gradual addition of the Eudragit NE40D aqueous dispersion to the bowl of a high shear granulator (GMX Micro). The granules were screened and dried in a tray dryer at 25°C for 12-16 hours and the granulation was repeated with the remaining portion of the Eudragit NE40D. The subsequent granules were again screened and dried in a tray dryer at 25°C for 12-16 hours.

The polyethylene oxide was added as an extragranular component along with a glidant (colloidal silicon dioxide) and a lubricant (magnesium stearate). The blend was compressed on a Pressima tablet press using multi-tip punches and dies to obtain tablets of 3 mm diameter with an average weight of 13 mg oxycodone HCl per tablet. In formulation 3A, 62.5% of the total Eudragit NE was incorporated in the first granulation step, and the remainder 37.5% was incorporated in the second granulation step.

A portion of the tablets were cured at 70°C for 15 minutes in a tray dryer. Cured and uncured tablets were divided in portions of 200 ± 5 mg into size 0 gelatin capsules with or without lactose (ranging from about 240 mg to about 270 mg) to obtain a dose of oxycodone HCl of 33.4 mg per capsule for formulation 3A and a dose of oxycodone HCl of 34.8 mg per capsule for formulation 3B (approximately 16 of the 3 mm tablets).

For all formulations, potency adjustments were not performed for taking into account the moisture content of 4-6% in the drug substances.

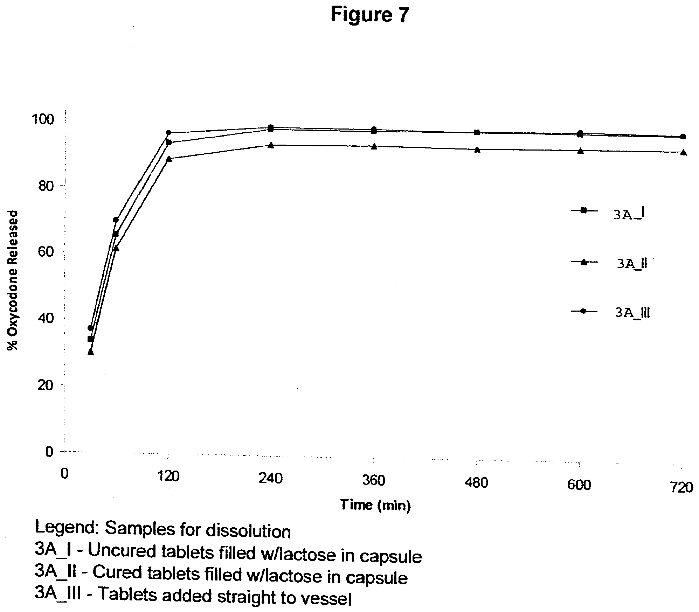

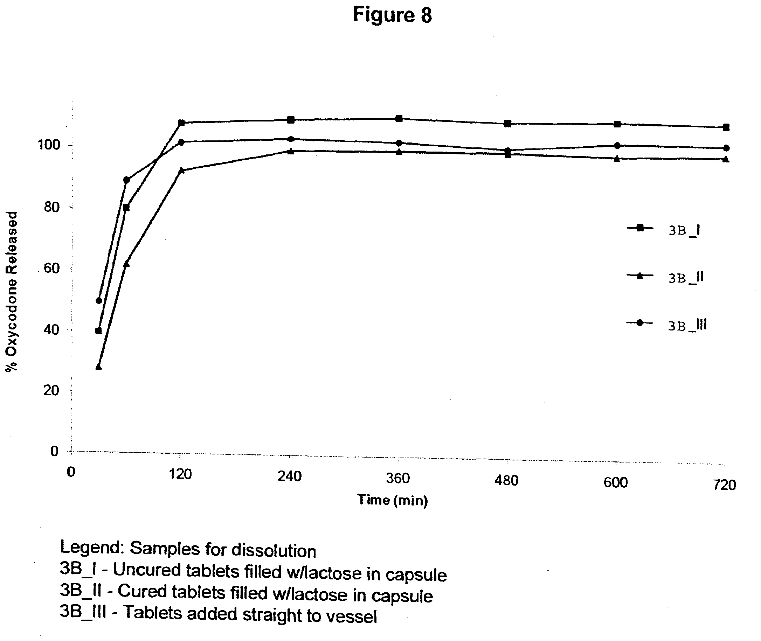

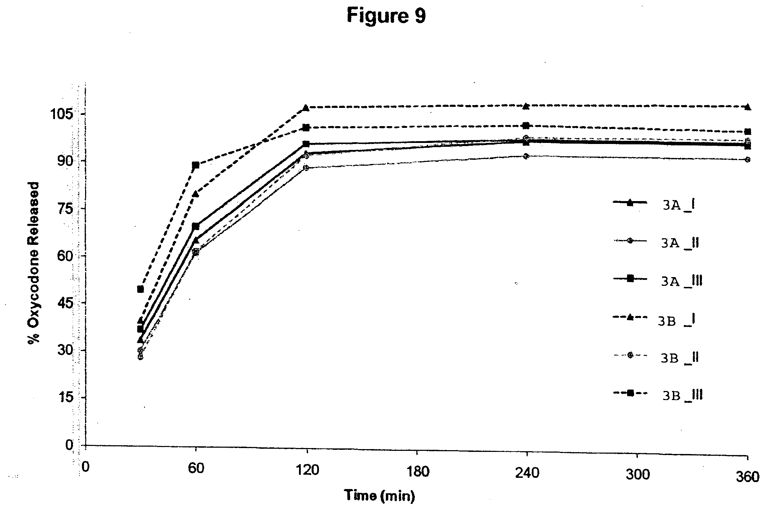

Cured and uncured formulations of Example 3 were subjected to dissolution testing in simulated gastric fluid without enzymes at a pH of 1.2 using a USP Apparatus 1 with a #10 mesh basket at 100 rpm or directly added to the vessel. The results are set forth in Figures 7-9.

### Reference Example 4A

Cores for extended release minitablets comprising oxycodone, a gelling agent and a neutral acrylic polymer were prepared in accordance with Table 4A:

**TABLE 4A**

| **Portion** | **Ingredient** | **Amount (% w/w)** |
|---|---|---|
| | | |
| Extragranular | Oxycodone HCl | 8.67 |
| | Eudragit NE | 30.7 |
| | Microcrystalline cellulose (MCC) | 19.63 |
| | Methyl Cellulose | 40.0 |
| | Magnesium Stearate | 0.5 |
| | Collodial Silicon Dioxide | 0.5 |
| | Total | 100 |

Processing of the cores included wet granulation of oxycodone HCl and microcrystalline cellulose with Eudragit NE 40D aqueous dispersion. The Eudragit® NE40D aqueous dispersion was incorporated into the granulation in three steps to increase the amount of solid NE. In formulation 4A, 58.7% of the total Eudragit NE was incorporated in the first granulation step, 21.2% of the total Eudragit NE was incorporated in the second granulation step, and the remainder 20.1% was incorporated in the third granulation step.

Each step of the wet granulation was performed by gradual addition of the Eudragit NE40D aqueous dispersion to the bowl of a high shear granulator (GMX Micro). The granules were screened and dried in a fluid bed processor as per the following Table 4.1 below:

**Table 4.1**

| Step # | Temperature (°C) | Time (h) |
|---|---|---|
| 1 | 25 | 2.25 |
| 2 | 40 | 1 |
| 3 | 40 | 1 |

The blend was prepared by adding methyl cellulose, magnesium stearate and colloidal silicon dioxide to the granules and compressed on a Pressima tablet press using multi-tip punches and dies to obtain tablets of 4 mm diameter with an average weight of 2.6 mg and oxycodone HCl per tablet of 30mg.

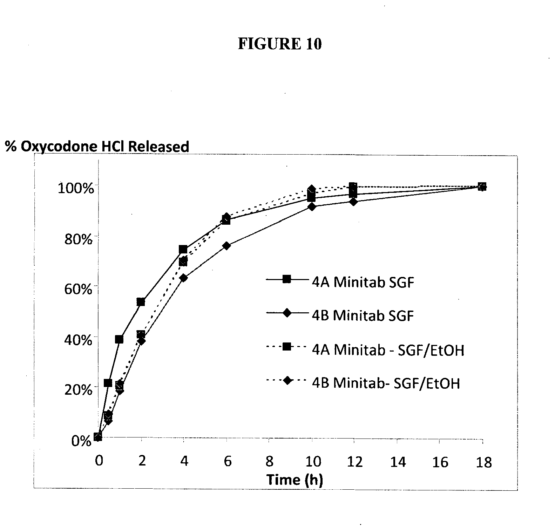

The formulation of Example 4A was subjected to dissolution testing in simulated gastric fluid without enzymes, and in simulated gastric fluid with 40% ethanol (i.e. 540 ml of SGF pH 1.2 mixed with 360 ml of 200 proof Ethanol), at a pH of 1.2 using USP Apparatus 2 (paddles) at 50 rpm. The results are set forth in Figure 10.

### Reference Example 4B

Cores for extended release minitablets comprising oxycodone, a gelling agent and a neutral acrylic polymer were prepared in accordance with Table 4B:

**TABLE 4B**

| **Portion** | **Ingredient** | **Amount (% w/w)** |
|---|---|---|
| | | |
| Intragranular | Oxy HCl | 8.67 |
| | Microcrystalline cellulose (MCC) | 19.63 |
| | Eudragit-NE solids | 30.7 |
| | Intragranular Total | 59.0 |
| Extragranular | Methyl Cellulose | 40.0 |
| | Magnesium Stearate | 0.5 |
| | Collodial Silicon Dioxide | 0.5 |
| | Total | 100 |

Processing of the cores included wet granulation of oxycodone HCl and microcrystalline cellulose with Eudragit NE 40D aqueous dispersion via granulation in four steps to increase the amount of solid NE. In formulation 4B, 48.1% of the total Eudragit NE was incorporated in the first granulation step, 20.2% of the total Eudragit NE was incorporated in the second granulation step, 17.4% was incorporated in the third granulation step, and the remainder 14.4% was incorporated in the fourth granulation step.

Each step of the wet granulation was performed by gradual addition of the Eudragit NE40D/methyl cellulose aqueous dispersion to the bowl of a high shear granulator (GMX Micro). The granules were screened and dried in a fluid bed processor as per the following Table 4.2 below.

**Table 4.2**

| Step # | Temperature Range (°C) | Time (h) |
|---|---|---|
| 1 | 19 - 24 | 4.5 |
| 2 | 30 - 35 | 0.75 |
| 3 | 30 - 35 | 0.75 |
| 4 | 30 - 35 | 0.75 |

The blend was prepared by adding methyl cellulose, magnesium stearate and colloidal silicon dioxide to the granules and compressed on a Pressima tablet press using multi-tip punches and dies to obtain tablets of 4 mm diameter with an average weight of 2.6 mg and oxycodone HCl per tablet of 30 mg.

The formulation of Example 4B was subjected to dissolution testing in simulated gastric fluid without enzymes, and in simulated gastric fluid (SGF) with 40% ethanol (i.e. 540 ml of SGF pH 1.2 mixed with 360 ml of 200 proof Ethanol), at a pH of 1.2 using USP Apparatus 2 (paddles) at 50 rpm. The results are set forth in Figure 10.

### Reference Example 4C

Cores for extended release minitablets comprising oxycodone, a gelling agent and a neutral acrylic polymer were prepared in accordance with Table 4C:

**TABLE 4C**

| **Portion** | **Ingredient** | **Amount (% w/w)** |
|---|---|---|
| | | |
| Extragranular | Oxycodone HCl | 31.89 |
| | Eudragit NE | 27.11 |
| | Methyl Cellulose | 40.0 |
| | Magnesium Stearate | 0.5 |
| | Collodial Silicon Dioxide | 0.5 |
| | Total | 100 |

Processing of the cores included wet granulation of oxycodone HCl with Eudragit NE 40D aqueous dispersion in a high shear granulator (GMX Micro). 45.5% of the total Eudragit NE was incorporated in the first granulation step, 23.4% of the total Eudragit NE was incorporated in the second granulation step, 18.5% was incorporated in the third granulation step, and the remainder 12.6% was incorporated in the fourth granulation step. The granules were screened and dried in a fluid bed processor as per the following Table 4.3 below.

**Table 4.3**

| Step # | Temperature Range (°C) | Time (h) |
|---|---|---|
| 1 | 25 | 1.5 |
| 2 | 35 - 40 | 2.25 |
| 3 | 30 - 42 | 1.5 |
| 4 | 33-37 | 1.5 |

The final Eudragit NE content was 46% in the granules.

The blend was prepared by adding methyl cellulose, magnesium stearate and colloidal silicon dioxide to the granules and compressed on a Pressima tablet press using multi-tip punches and dies to obtain tablets of 4 mm diameter with an average weight of 9.57 mg and oxycodone HCl per tablet of 30 mg.

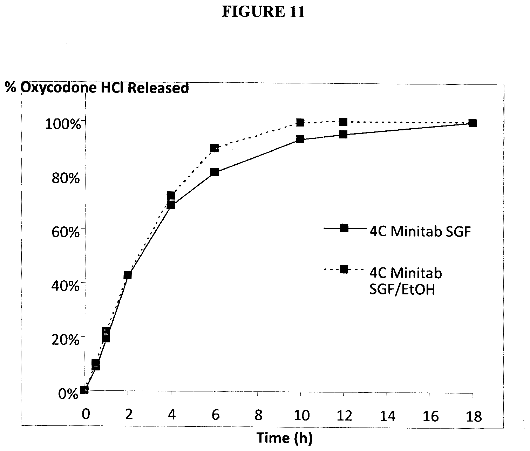

The formulation of Example 4C was subjected to dissolution testing in simulated gastric fluid without enzymes, and in simulated gastric fluid (SGF) with 40% ethanol (i.e. 540 ml of SGF pH 1.2 mixed with 360 ml of 200 proof Ethanol), at a pH of 1.2 using USP Apparatus 2 (paddles) at 50 rpm. The results are set forth in Figure 11.

### Reference Example 4D

Cores for biphasic release minitablets comprising oxycodone and a gelling agent and a neutral acrylic polymer were prepared in accordance with Table 4D:

**TABLE 4D:**

| **Portion** | **Ingredient** | **Amount (% w/w)** |
|---|---|---|
| | | |
| Extragranular | Oxycodone HCl | 8.62 |
| | Eudragit NE | 34.43 |
| | Microcrystalline Cellulose | 15.95 |
| | Methyl Cellulose | 40.0 |
| | Magnesium Stearate | 0.5 |
| | Collodial Silicon Dioxide | 0.5 |
| | Total | 100 |

The granules formed in granulation step of Example 4C were mixed with microcrystalline cellulose in a 1:1 w/w ratio in a high shear granulator (GMX Micro) and granulated with additional Eudragit NE 40D dispersion for a final Eudragit NE content of 58% in the granules. 21.3% of total Eudragit NE was carried over from granules of Example 4C. The additional NE was added as 40% of the total Eudragit NE was incorporated in the first granulation step, 20% of the total Eudragit NE was incorporated in the second granulation step, and the remainder 18.7% was incorporated in the third granulation step.

The blend was prepared by adding methyl cellulose, magnesium stearate and colloidal silicon dioxide to the granules and compressed on a Pressima tablet press using multi-tip punches and dies to obtain tablets of 4 mm diameter with an average weight of 2.59 mg and oxycodone HCl per tablet of 30 mg.

The formulation of Example 4D was subjected to dissolution testing in simulated gastric fluid without enzymes at a pH of 1.2 using a USP Apparatus 1 with a #10 mesh basket at 100 rpm or directly added to the vessel. The results are set forth in Figure 12.

### Reference Example 5

Cores for immediate release minitablets comprising oxycodone and a gelling agent and a neutral acrylic polymer were prepared in accordance with Table 5:

**TABLE 5**

| **Portion** | **Ingredient** | **Amount (% w/w)** | | | |
|---|---|---|---|---|---|
| | | **5A** | **5B** | **5C** | **5D** |
| Extragranular | Oxycodone HCl | 16.07 | 12.81 | 16.07 | 12.81 |
| | Eudragit NE | 36.37 | 29.0 | 36.37 | 29.0 |
| | Microcrystalline Cellulose (MCC) | 21.56 | 17.19 | 21.56 | 17.19 |
| | Methyl Cellulose A4M | 25.0 | 40.0 | - | - |
| | Methyl Cellulose A40M | - | - | 25.0 | 40.0 |
| | Magnesium Stearate | 0.5 | 0.5 | 0.5 | 0.5 |
| | Collodial Silicon Dioxide | 0.5 | 0.5 | 0.5 | 0.5 |
| | Total | 100 | 100 | 100 | 100 |

Processing of the cores included dissolving of oxycodone HCl in Eudragit NE 40D aqueous dispersion. The mixture of Oxycodone HCl -NE was sprayed onto MCC in a high shear granulator (GMX Micro). 54.9% of the total Eudragit NE was incorporated in the first granulation step, 21.4% of the total Eudragit NE was incorporated in the second granulation step, 13.2% was incorporated in the third granulation step, and the remainder 10.4% was incorporated in the fourth granulation step. The final Eudragit NE content was 49%

The blend was compressed on a Pressima tablet press using multi-tip punches and dies to obtain tablets of 4 mm diameter with an average weight of 30 mg. The minitablets contained 4.82 mg oxycodone HCl/tablet for 25 % methyl cellulose tablet (formulations 5A and 5C), and 3.84 mg oxycodone HCl/tablet per 40% methylcellulose tablet (formulations 5B and 5D).

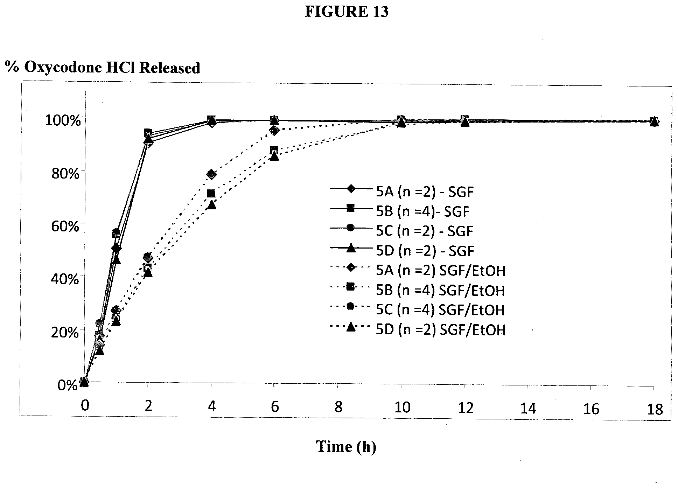

The formulation of Examples 5A, B, C and D were subjected to dissolution testing in simulated gastric fluid without enzymes, and in simulated gastric fluid (SGF) with 40% ethanol (i.e. 540 ml of SGF pH 1.2 mixed with 360 ml of 200 proof Ethanol), at a pH of 1.2 using USP Apparatus 2 (paddles) at 50 rpm. The results are set forth in Figure 13.

### Reference Example 6

Cores for immediate release minitablets comprising oxycodone and a gelling agent and a neutral acrylic polymer were prepared in accordance with Table 6:

**TABLE 6**

| **Portion** | **Ingredient** | **Amount (% w/w)** |
|---|---|---|
| | | |
| Intragranular | Oxycodone HCl | 8.66 |
| | Eudragit NE | 30.69 |
| | Microcrystalline Cellulose (MCC) | 19.64 |
| | Methyl Cellulose A40M | 40.01 |
| | Magnesium Stearate | 0.5 |
| | Collodial Silicon Dioxide | 0.5 |
| | Total | 100 |

Processing of the cores included wet granulation of oxycodone HCl with microcrystalline cellulose, methyl cellulose and Eudragit NE 40D aqueous dispersion in a high shear granulator (GMX Micro). The granules were screened and dried in a fluid bed processor with an inlet temperature range of 25 to 40°C for 48 minutes. The final Eudragit NE content was 30.7%.

The blend was compressed on a Pressima tablet press using multi-tip punches and dies to obtain tablets of 4 mm diameter with an average weight of 2.6 mg and oxycodone HCl per tablet of 30 mg.

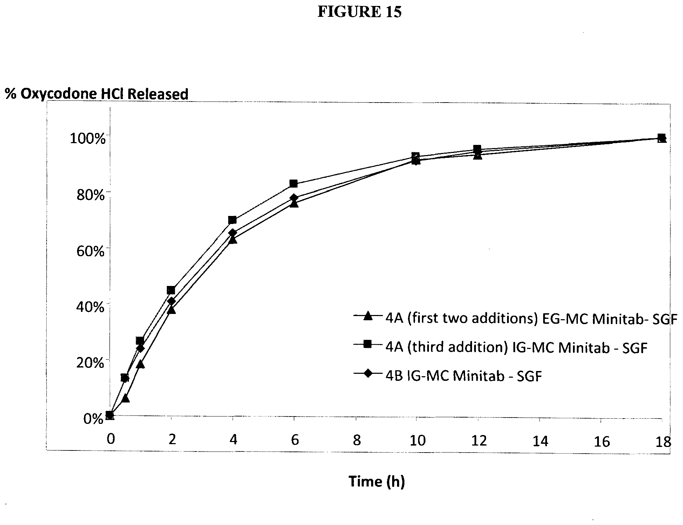

The formulation of Example 6 was subjected to dissolution testing in simulated gastric fluid without enzymes at a pH of 1.2 using USP Apparatus 2 (paddles) at 50 rpm. The results are set forth in Figure 15.

### Reference Example 7

Cores for immediate release minitablets comprising oxycodone and a gelling agent and a neutral acrylic polymer were prepared in accordance with Table 7:

**TABLE 7**

| **Portion** | **Ingredient** | **Amount (% w/w)** |
|---|---|---|
| | | |
| Intragranular | Oxycodone HCl | 7.53 |
| | Eudragit NE | 39.60 |
| | Microcrystalline Cellulose (MCC) | 17.08 |
| | Methyl Cellulose A40M | 34.79 |
| | Magnesium Stearate | 0.5 |
| | Collodial Silicon Dioxide | 0.5 |
| | Total | 100 |

The granules formed in granulation step of Example 6 were granulated with additional Eudragit NE 40D dispersion for a final Eudragit NE content of 40%.

The blend was compressed on a Pressima tablet press using multi-tip punches and dies to obtain tablets of 4 mm diameter with an average weight of 2.6 mg and oxycodone HCl per tablet of 30 mg.

The formulation of Example 7 was subjected to dissolution testing in simulated gastric fluid without enzymes at a pH of 1.2 using USP Apparatus 2 (paddles) at 50 rpm. The results are set forth in Figure 15.

### Example 8

The formulations of Examples 4 and 5 were subjected to qualitative tamper testing for syringeability. The minitablets were crushed and dispersed in 5ml purified water at room temperature for 10 minutes, then aspirated through syringes fitted with needles of gauges 18", 22", 25" and 27". The results are shown in Table 8 below:

**Table 8**

| **Sample** | **#18** | **#22** | **#25** | **#27** | **Comments** |
|---|---|---|---|---|---|
| Process 1 PEO 301 - 25% | - | -- | x | x | Very viscous, translucent gel |
| Process 2 PEO 301 - 25% | - | -- | x | x | Very viscous, clumped translucent gel |
| Process 2 MC4M-40% | + | -- | -- | x | Very viscous, clumped translucent gel |
| Process 3A MC40M - 40% | - | - | -- | -- | Very Viscous |
| Process 3B PEO 301 - 25% | ++ | - | - | -- | Slightly more viscous |
| Process 3A MC40M - 25% | ++ | ++ | - | -- | Viscous gel |
| Process 2 MC4M - 25% | ++ | + | - | -- | Viscous gel |
| Process 2 MC40M - 25% | ++ | + | - | -- | Slightly viscous gel |
| Process 2 PEO 301 - 25% | ++ | ++ | - | - | Slight Viscosity Change |
| Process 3A MC4M - 40% | ++ | ++ | ++ | ++ | Some viscosity floating granules |
| Process 2 PEO 301 - 25% | ++ | ++ | ++ | ++ | No Viscosity Change |

### Description

++ Very easy to aspirate
+ Easy to aspirate
- Difficult to aspirate
-- Very difficult to aspirate
x Could not be aspirated

### Example 9

The formulations of Examples 4A and 4B, and OxyContin™ for control, were crushed in a mortar and pestle for 60 seconds and 30 seconds, respectively, and then subjected to dissolution testing in simulated gastric fluid without enzymes at a pH of 1.2 using a USP Apparatus 1 with a #40 mesh basket at 100 rpm.

## Claims

1. An oral dosage form comprising from 2 to 75 particles, each particle comprising a compressed core comprising:
(i) an active agent susceptible to abuse;
(ii) a gelling agent comprising polyethylene oxide; and
(iii) a neutral acrylic polymer;
wherein the 2 to 75 particles contain a therapeutically or prophylactically effective amount of the active agent; and
wherein the viscosity resulting from mixing an intact unit dose of the dosage form with from 0.5 to 10 ml of an aqueous liquid is at least 10 mPa s (10 cP).

2. The oral dosage form of claim 1, which provides an immediate release of the active agent.

3. The oral dosage form of claim 1, which provides a controlled release of the active agent.

4. The oral dosage form of claim 1, wherein the amount of active agent at 0.5 hour when measured in a USP Apparatus 1 (basket) at 100 rpm in 900 ml simulated gastric fluid (SGF) without enzymes with 40% ethanol at 37° C, is within 20% of the amount of active agent released at 0.5 hour when measured in a USP Apparatus 1 (basket) at 100 rpm in 900 ml simulated gastric fluid without enzymes (SGF) with 0% ethanol at 37°C.

5. The oral dosage form of claim 1, wherein the amount of gelling agent is less than the amount of drug.

6. The oral dosage form of claim 1, wherein the amount of gelling agent is more than the amount of drug.

7. The oral dosage form of claim 1, wherein the weight ratio of gelling agent to drug is from 5:1 to 1:5.

8. The oral dosage form of claim 1, wherein the gelling agent further comprises an agent selected from the group consisting of sugars, sugar derived alcohols, starch, starch derivatives, cellulose derivatives, attapulgites, bentonites, dextrins, alginates, carrageenan, gum tragacanth, gum acacia, guar gum, xanthan gum, pectin, gelatin, kaolin, lecithin, magnesium aluminum silicate, carbomers, carbopols, polyvinylpyrrolidone, polyethylene glycol, polaxamers, polycarbophil, polyvinyl alcohol, silicon dioxide, surfactants, mixed surfactant/wetting agent systems, emulsifiers, and mixtures thereof.

9. The oral dosage form of claim 8, wherein the gelling agent further comprises an agent selected from the group consisting of sugars, sugar derived alcohols, cellulose derivatives, gums, polymers, and mixtures thereof.

10. The oral dosage form of claim 1, wherein the active agent is selected from the group consisting of an opioid agonist, a tranquilizer, a CNS depressant, a CNS stimulant, a sedative hypnotic, and mixtures thereof.

11. The oral dosage form of claim 10, wherein the drug is an opioid agonist.

12. The oral dosage form of claim 11, wherein the opioid agonist is selected from the group consisting of codeine, morphine, oxycodone, oxymorphone, hydrocodone, hydromorphone, pharmaceutically acceptable salts thereof, and mixtures thereof.

13. The oral dosage form of claim 2, wherein the dosage form releases at least 85% of the drug within 45 minutes as measured by in-vitro dissolution in a USP Apparatus 1 (basket) at 100 rpm in 900 ml simulated gastric fluid (SGF) without enzymes at 37° C.

14. The oral dosage form of claim 3, which provides a dissolution release rate in-vitro of the active agent, when measured by the USP Basket Method at 100 rpm in 700 ml Simulated Gastric Fluid (SGF) without enzymes at 37° C of at least 15% by weight of the active agent released at 1 hour and thereafter switching to 900 ml with Phosphate Buffer at a pH of 7.5 at 37° C, of from 25% to 65% by weight of the active agent released at 2 hours, from 45% to 85% by weight of the active agent released at 4 hours, and at least 60% by weight of the active agent released at 8 hours.

15. The oral dosage form of claim 1, wherein the 2 to 75 particles are contained in a pharmaceutically acceptable capsule.

## Patentansprüche

1. Orale Darreichungsform, umfassend 2 bis 75 Teilchen, wobei jedes Teilchen einen komprimierten Kern umfasst, umfassend:
(i) einen missbrauchsanfälligen Wirkstoff;
(ii) ein Geliermittel, das Polyethylenoxid umfasst; und
(iii) ein neutrales Acrylpolymer;
wobei die 2 bis 75 Teilchen eine therapeutisch oder prophylaktisch wirksame Menge des Wirkstoffs enthalten; und
wobei die Viskosität, die sich aus dem Mischen einer intakten Einheitsdosis der Darreichungsform mit 0,5 bis 10 ml einer wässrigen Flüssigkeit ergibt, mindestens 10 mPa s (10 cP) beträgt.

2. Orale Darreichungsform nach Anspruch 1, die eine sofortige Freisetzung des Wirkstoffs bereitstellt.

3. Orale Darreichungsform nach Anspruch 1, die eine kontrollierte Freisetzung des Wirkstoffs bereitstellt.

4. Orale Darreichungsform nach Anspruch 1, wobei die Menge an Wirkstoff nach 0,5 Stunden, gemessen in einer USP-Apparatur 1 (Korb) bei 100 U/min in 900 ml simulierter Magenflüssigkeit (SGF) ohne Enzyme mit 40 % Ethanol bei 37 °C, innerhalb von 20 % der Wirkstoffmenge liegt, die bei 0,5 Stunden freigesetzt wird, gemessen in einer USP-Apparatur 1 (Korb) bei 100 U/min in 900 ml simulierter Magenflüssigkeit (SGF) ohne Enzyme mit 0 % Ethanol bei 37 °C.

5. Orale Darreichungsform nach Anspruch 1, wobei die Menge an Geliermittel weniger als die Menge an Arzneistoff ist.

6. Orale Darreichungsform nach Anspruch 1, wobei die Menge an Geliermittel mehr als die Menge an Arzneistoff ist.

7. Orale Darreichungsform nach Anspruch 1, wobei das Gewichtsverhältnis von Geliermittel zu Arzneistoff 5:1 bis 1:5 beträgt.

8. Orale Darreichungsform nach Anspruch 1, wobei das Geliermittel ferner ein Mittel umfasst, ausgewählt aus der Gruppe bestehend aus Zuckern, von Zucker abgeleiteten Alkoholen, Stärke, Stärkederivaten, Cellulosederivaten, Attapulgiten, Bentoniten, Dextrinen, Alginaten, Carrageenan, Tragantgummi, Akaziengummi, Guargummi, Xanthangummi, Pektin, Gelatine, Kaolin, Lecithin, Magnesiumaluminiumsilikat, Carbomeren, Carbopolen, Polyvinylpyrrolidon, Polyethylenglykol, Polaxameren, Polycarbophil, Polyvinylalkohol, Siliciumdioxid, Tensiden, Misch-Tensid/Netzmittelsystemen, Emulgatoren und Gemischen davon.

9. Orale Darreichungsform nach Anspruch 8, wobei das Geliermittel ferner ein Mittel umfasst, ausgewählt aus der Gruppe bestehend aus Zuckern, von Zucker abgeleiteten Alkoholen, Cellulosederivaten, Gummis, Polymeren und Gemischen davon.

10. Orale Darreichungsform nach Anspruch 1, wobei der Wirkstoff aus der Gruppe bestehend aus einem Opioid-Agonisten, einem Tranquilizer, einem ZNS-Beruhigungsmittel, einem ZNS-Stimulans, einem Beruhigungshypnotikum und Gemischen davon ausgewählt ist.

11. Orale Darreichungsform nach Anspruch 10, wobei der Arzneistoff ein Opioid-Agonist ist.

12. Orale Darreichungsform nach Anspruch 11, wobei der Opioid-Agonist aus der Gruppe bestehend aus Codein, Morphin, Oxycodon, Oxymorphon, Hydrocodon, Hydromorphon, pharmazeutisch annehmbaren Salzen davon und Gemischen davon ausgewählt ist.

13. Orale Darreichungsform nach Anspruch 2, wobei die Darreichungsform mindestens 85 % des Arzneistoffs innerhalb von 45 Minuten freisetzt, gemessen durch In-vitro-Auflösung in einer USP-Apparatur 1 (Korb) bei 100 U/min in 900 ml simulierter Magenflüssigkeit (SGF) ohne Enzyme bei 37 °C.

14. Orale Darreichungsform nach Anspruch 3, die eine In-vitro-Auflösungsfreisetzungsrate des Wirkstoffs, gemessen nach dem USP-Korbverfahren bei 100 U/min in 700 ml simulierter Magenflüssigkeit (SGF) ohne Enzyme bei 37 °C, von mindestens 15 Gew.-% nach 1 Stunde freigesetztem Wirkstoff, und nach anschließendem Wechseln zu 900 ml mit Phosphatpuffer bei einem pH-Wert von 7,5 bei 37 °C, von 25 bis 65 Gew.-% nach 2 Stunden freigesetztem Wirkstoff, von 45 bis 85 Gew.-% nach 4 Stunden freigesetztem Wirkstoff und mindestens 60 Gew.-% nach 8 Stunden freigesetztem Wirkstoff bereitstellt.

15. Orale Darreichungsform nach Anspruch 1, wobei die 2 bis 75 Teilchen in einer pharmazeutisch annehmbaren Kapsel enthalten sind.

## Revendications

1. Forme galénique orale comprenant de 2 à 75 particules, chaque particule comprenant un noyau comprimé, comprenant :
(i) un agent actif pouvant être à l'origine d'une pharmacodépendance ;
(ii) un agent gélifiant comprenant de l'oxyde de polyéthylène ; et
(iii) un polymère acrylique neutre ;
dans lequel les 2 à 75 particules contiennent une quantité thérapeutiquement ou prophylactiquement efficace de l'agent actif ; et
dans lequel la viscosité obtenue en mélangeant une dose unitaire intacte de la forme galénique avec de 0,5 à 10 ml d'un liquide aqueux est d'au moins 10 mPas (10 cP).

2. Forme galénique orale selon la revendication 1, qui procure une libération immédiate de l'agent actif.

3. Forme galénique orale selon la revendication 1, qui procure une libération contrôlée de l'agent actif.

4. Forme galénique orale selon la revendication 1, dans laquelle la quantité d'agent actif à 0,5 h lorsque mesurée dans un appareil USP 1 (panier) à 100 tpm dans 900 ml de fluide gastrique simulé (SGF) en absence d'enzymes avec de l'éthanol à 40 % à 37 °C, est dans les 20 % de la quantité d'agent actif libéré à 0,5 h lorsque mesurée dans un appareil USP 1 (panier) à 100 tpm dans 900 ml de fluide gastrique simulé (SGF) en absence d'enzyme avec de l'éthanol à0 % à 37 °C.

5. Forme galénique orale selon la revendication 1, dans laquelle la quantité d'agent gélifiant est inférieure à la quantité de médicament.

6. Forme galénique orale selon la revendication 1, dans laquelle la quantité d'agent gélifiant est supérieure à la quantité de médicament.

7. Forme galénique orale selon la revendication 1, dans laquelle le rapport pondéral de l'agent gélifiant sur le médicament est de 5:1 à 1:5.

8. Forme galénique orale selon la revendication 1, dans laquelle l'agent gélifiant comprend un agent choisi dans le groupe constitué de sucres, d'alcools dérivés de sucre, d'amidon, de dérivés d'amidon, de dérivés de cellulose, d'attapulgites, de bentonites, de dextrines, d'alginates, du carraghénane, de gomme adragante, de gomme arabique, de gomme de guar, de gomme de xanthane, de pectine, de gélatine, du kaolin, de lécithine, du silicate de magnésium et d'aluminium, de carbomères, de carbopols, du polyvinylpyrrolidone, du polyéthylène glycol, de polaxamères, de polycarbophile, de l'alcool polyvinylique, du dioxyde de silicone, d'agents tensio-actifs, de systèmes mixtes agent tensio-actif/agent mouillant, d'émulsifiants, et des mélanges de ceux-ci.

9. Forme galénique orale selon la revendication 8, dans laquelle l'agent gélifiant comprend un agent choisi dans le groupe constitué de sucres, d'alcools dérivés de sucre, de dérivés de cellulose, de gommes, de polymères, et des mélanges de ceux-ci.

10. Forme galénique orale selon la revendication 1, dans laquelle l'agent actif est choisi dans le groupe constitué d'un agoniste d'opioïde, d'un tranquillisant, d'un déprimant du SNC, d'un stimulant du SNC, d'un hypnotique sédatif, et des mélanges de ceux-ci.

11. Forme galénique orale selon la revendication 10, dans laquelle le médicament est un agoniste d'opioïde.

12. Forme galénique orale selon la revendication 11, dans laquelle l'agoniste d'opioïde est choisi dans le groupe constitué de la codéine, de la morphine, de l'oxycodone, de l'oxymorphone, de l'hydrocodone, de l'hydromorphone, des sels pharmaceutiquement acceptables de ceux-ci, et des mélanges de ceux-ci.

13. Forme galénique orale selon la revendication 2, dans laquelle la forme galénique libère au moins 85 % du médicament dans les 45 minutes tel que mesuré par la dissolution in-vitro dans un appareil USP 1 (panier) à 100 tpm dans 900 ml de fluide gastrique simulé (SGF) en absence d'enzymes à 37 °C.

14. Forme galénique orale selon la revendication 3, qui procure une vitesse de libération par dissolution in-vitro de l'agent actif, mesurée par la méthode du panier USP à 100 tpm dans 700 ml de fluide gastrique simulé (SGF) en absence d'enzymes à 37 °C d'au moins 15 % en poids de l'agent actif libéré à 1 h et ensuite en passant à 900 ml avec du tampon phosphaté à un pH de 7,5 à 37 °C, de 25 % à 65 % en poids de l'agent actif libéré à 2 h, de 45 % à 85 % en poids de l'agent actif libéré à 4 h, et au moins 60 % en poids de l'agent actif libéré à 8 h.

15. Forme galénique orale selon la revendication 1, dans laquelle les 2 à 75 particules sont contenues dans une capsule pharmaceutiquement acceptable.
